# EUROPEAN PATENT SPECIFICATION

(11) **EP 1 644 507 B1**
(45) Date of publication and mention of the grant of the patent: **17.03.2010**
(21) Application number: 04742169.8
(22) Date of filing: 05.07.2004
(51) Int. Cl.: C12N 15/82

(54) **NOVEL RUBISCO PROMOTERS AND USES THEREOF**
NEUE RUBISCO-PROMOTOREN UND VERWENDUNGEN DAVON
NOUVEAUX PROMOTEURS DE LA RUBISCO ET LEURS UTILISATIONS

(30) Priority: 03.07.2003 US 484707 P; 10.07.2003 FI 20031052; 02.07.2004 US 884283
(43) Date of publication of application: 12.04.2006
(73) Proprietor: Unicrop Oy, 00790 Helsinki (FI)
(72) Inventor: ANISSIMOV, Andrei, FIN-00790 Helsinki (FI); KAIJALAINEN, Seppo, FIN-00980 Helsinki (FI); KOIVU, Kimmo, FIN-01100 Itäsalmi (FI); JUNTUNEN, Kari, FIN-02680 Espoo (FI); KANERVA, Anne, FIN-01100 Itäsalmi (FI)
(74) Representative: Lönnqvist, Gunnel Solveig Kristina
(86) International application number: PCT/FI2004/000426
(87) International publication number: WO 2005/003360

(56) References cited:
- WO-A1-01/41559
- US-A1- 2002 170 096
- FIEBIG C. ET AL.: '5'-upstream cis-elements and binding factor(s) potentially involved in light-regulated expression of a Brassica napus rbcS gene' CURR. GENET. vol. 21, 1992, pages 161 - 168, XP002903837
- FIEBIG C. ET AL.: 'Sequence characteristics and transcripts of rbcS genes from Brassica napus: Temporal and spatial expression during crucifer seedling morphogenesis' BOT. ACTA vol. 103, 1990, pages 258 - 265, XP002903838
- BECK ET AL: "Transient expression activity of RbcS promoter regions from Brassica napus in mesophyll protoplasts from Nicotiana tabacum" vol. 108, pages 327-333, XP009084139

### SEQUENCE LISTING

<110> UniCrop Ltd
<120> A Rubisco Promoter and the Use Thereof
<130> A3542PC
<150> FI 20031052
   <151> 2003-07-10
<150> US 60/484,707
   <151> 2003-07-03
<150> US
   <151> 2004-07-02
<160> 32
<170> PatentIn version 3.2
<210> 1
   <211> 2175
   <212> DNA
   <213> Brassica rapa
<400> 1
   gtttcctgag tgttgcacct cctcagtctg agccgtatta agttcaaacc aaactgccgc     60
   ttctgctcta gctttactca ggactgaaac cgaggagtag tgtatcttct caaaagccag    120
   accatttctc gccttccata gatgccagag tatccaagga aaaaacatcg tatcgctatc    180
   ttttggagac ttcctactca tttccaggag atgatataaa cctgccaccg ggagtttgat    240
   cgaggacctt tcccaagtgt ccttcgccat ggggcaagaa aaaagtagat ggcagatcga    300
   ttcaatacca ccattacata ctttgcaagc agagtctact tgaatacctc tactacgaag    360
   acgttccatg accgctaaag ccccagagag agctttccaa aggaaatgct taatttttgg    420
   aggagcacgg atcttccaca gagatttcca aaggtatttc tccaggggag gaagagaagt    480
   tgaaatgcta ccatcttcct caggaagcga atcgacaaat ctttaaccac tccgtgatgt    540
   gtagattcca tcttttgtga agccccactt ataaccatct tcctgcatcc tatttggctt    600
   caagcgaagg atgatctcag catcatgatc agtaaacgtt cttctcacaa gagctgcatc    660
   ccaggcagaa gagttaggga gcagcaggtc tgagattgtc agcgtcagat caatgacact    720
   atcctgtctg tagttaggag tcctcggtat agggtcgata atccaattca cgtgccacac    780
   attagaactc ctaccattac caatgtctct aatcaaacct ttgctcagca gctctctacc    840
   atgtaaaatg cttcgccatg catatgacgg cctcgaacct aaactgcttt gcaagaagtc    900
   attgtttgca aagtatctac ttttgaggat tcgagccacc agagagttgg ggttattcat    960
   gatcctccat gcttgcttag ccaagaaagt taagattagt gataacgatc agctagcgaa   1020
   gaaagttaag attagtgatt agaactacgt aatcacctgt cacatttagc tggccttttc   1080
   tgtattctaa tattttttaa aatgaaatta tcaacagaaa aaagatattt taaattctta   1140
   ataaaatgaa attatttttc tgacccgctc tggccttgac ctctataaat atttgagccg   1200
   gtgctatgtt caaagttctt ttcggtcagc ttgcgtctgc atagtgcata tgatggaagg   1260
   attttttggt ggtacatgct ctcggcggtg tagaagcttg cagaagagtt gatgaaattt 1320
   gagtcatcac tagcggattc agtgacggta atgaggtgat gacgtagctg aaccaattac 1380
   gcgtgacatt tcgtagggag acgcgtattg gtgatataaa ttcttaaaac tacaagtgtt 1440
   agagtatgtt taatagggag ttcttaaggt gtggttctta gaacatgatt atcgatggtg 1500
   ggaggagggt ttttacgcgt ggacccctcc atctttttgc aaacaccgtg tttcaatgtc 1560
   cccaaataaa aaatgtttca cagaggtttg ttgtactgtt tatggatctc acagacacgt 1620
   agcgacttgc gattcatttt tttttttttt ttttttaaat ttaagaaacc caaaagtgtt 1680
   tttaccgata atcatgctct tagttcttag cgtttttagt taaaagttaa aagacagatt 1740
   tttatattcc gttaaaaatc cttcattaag cctgcagtaa ttttcttttt tgtgtgcaac 1800
   tacaagtggt agcaaattaa tgtaactttc tttcacggcg ttgattgttg ggccgattat 1860
   gtaccacacg atctcatcct tatgggctct acgaaaagtt gacccacgaa aataaggaag 1920
   gagcctaaag catcggctca agtggagacc agaccagtaa ccatacgttt tcataatacg 1980
   ataagataag ataacgtttc tgtcacgtgg cattttcatt gtggtcaagt atcgagataa 2040
   gggtatcaac accgttcata atcctgtggc tgttaacgac gatatcatga aatatccata 2100
   agggttctca ctctatatag atgaccaaag caatagacta acagtaagag ttaagagaag 2160
   gaagaagaag tagtc 2175
<210> 2
   <211> 1016
   <212> DNA
   <213> Brassica rapa
<220>
   <221> misc_feature
   <222> (1)..(1016)
   <223> RbcS-4B
<400> 2
   aacccgaacc gaatccgatc cgataaaaat gaatccgaac cgatccgaat ccgacataaa 60
   taccgaatgg atcctgtttt ttggtatttt gggttatggg tattatccga accgaacccg 120
   gacctaaatg gatatccgat agaacccgaa acatttaaaa tcacaaaaag aacttctacc 180
   aaatatgatc ataattctta atatgtatcc aaaatacttt aagatattat tgaacttcta 240
   aaataattat atgttacatg aaggttgatg gtggaatgtg gcggttgatg cctgaagttt 300
   ttaggttttg gttttgtttt tattgaataa tgtttctcat ttcatgagaa cttatttttt 360
   gttttatgat ttcatttatc tggttttctt tctatcacta actatgttta tattttgctt 420
   gattttgaat gatcacgttt gatgtttttt cttatttttg aatcgatttt acttatgttt 480
   tggctattaa aatatgtaca aatcatgtat tttaaattcg aagaaccgat ttcatttatg 540
   ttttagttac aaaataggta caaatcagat atttttaaac caaagaaccg attgggaccc 600
   gaacccgaaa gtacaatgag ttataccggt tctttgaaga tttactaacc ccgacccgaa 660
   cccgatagaa cccgaaccgg tcccgaaccg aacttttata taacccgaat ggggttgatt 720
   ttgataaacc cgaaaaaccg aaacccgaat ggataaaacc gaaacccgat tgggaccccg 780
   aatgcccatg cctaccagta accatgcgtt ttcataatac gataagataa gataacgttt 840
   ctgtcacgtg gcattttcat tgtggtcaag tatcgagata agggtatcaa caccgttcat 900
   attcctgtgg ctgttaacga cgatatcatg aaatatccat aagggttctc actctatata 960
   gatgaccaaa gcaatagact aacagtaaga gttaagagaa ggaagaagaa gtagtc 1016
<210> 3
   <211> 1651
   <212> DNA
   <213> Brassica rapa
<220>
   <221> misc_feature
   <222> (1)..(1651)
   <223> RbcS-2
<400> 3
   cccttttccg tcataagttt tatatatata aaaacatatt tgcccttctt atctccctca 60
   tctctctctc tctctctctc tctctctctc tctctctctc tctctctctc tctctccctc 120
   tctctctctc tctctctctc tcatccggcg agtgtaaaca tatataacta ttaaattaat 180
   ttagaaaaca aactgtttaa tatagccaaa aatgaacgtg gggttttagt tgtaaaacaa 240
   aaaaaccata ctaactcaaa tctgaacaca ccgtattttt cttgatgttg aattaccact 300
   tgatttgtca attttaccaa aggaatccct caaaattgta actcaaatgt aatacattat 360
   acatcaaaaa gtaactcaaa aatctaaaaa ctaatcttac atttaaccca aacttaatct 420
   tataacacat tgcttttaat gtaagtatga ttttttggat taataacttt tacattcatt 480
   aaaaaaatca taaaaaatac aacactatct gaccagagct aaatataatt tgtaatcttg 540
   tatcattgcg atagaggcat ccgaaatttc atttaattga gaagttcggt tcggttcagc 600
   atgtttataa aaaaaatggt ttttggctcg ttctgttcgg taatcggtta gatcggtttc 660
   aaaaaaaatt tgttcccaaa tttcaatccg aactaaccta gctaaccaaa acttcgaatc 720
   gaagtaacca agctacctaa aattactcaa aattttgaac cgaactaaca gaattaacca 780
   aaacattgga ccgaattaac caattttacc caaattttta accgaaatat aatcagaacc 840
   aaaaacttaa gttagtttcg gtaaaatttt aaaaactgaa ctacccaaat accaaactga 900
   actgaatttt ttttatgttc gacaagattt tagtcgaacc gaactacctg aatccgcagg 960
   aagttaccca tcacagagag atgcacaaag cattacctaa aaacgttaca tttagtgttt 1020
   tggtaccact tttattgatt tttttttttg acagctattg atttagttta tagtttttaa 1080
   ctattaagta acagtttgtt tttcgtgtca aaaaaaaaag taacagtttt tatacggttt 1140
   tacttttaac ttaccaatcg gacccactat tcttttgttt ttgttggttt gaatatggac 1200
   atgaccatta cagtagtatc attactcata agttaattag tacgacatac atgtataatt 1260
   caagtacatc tcgtatagta atttcaattg tgaatttaat aatgaaccta atcaaattaa 1320
   gcgaaactaa ttcatataaa tagaaggtcc gcgaacattg aaatgtagat catgcgtcag 1380
   aattgtcctc tctcagtagg aaggagccaa aagcattggc tcaagttgag acgagtaacc 1440
   atacacattc atacgttttc ttacaagata agataagata atgttatttc tacacctttc 1500
   tttaatacct gtggcagtta acgacgatat catgaaatca tgatccttcg atcattaggg 1560
   ctttatacct cttgcgcttc tcactatata tagataacca aagcaataga caaacaagta 1620
   agttaagaga aaagaagaag aagaagtagt a 1651
<210> 4
   <211> 42
   <212> DNA
   <213> Artificial
<220>
   <223> primer e3a
<400> 4
   caucaucauc aucaaccgtc aagtccagtg catcagtttc at 42
<210> 5
   <211> 27
   <212> DNA
   <213> Artificial
<220>
   <223> primer atu
<400> 5
   cuacuacuac uatttttttt ttttttt 27
<210> 6
   <211> 48
   <212> DNA
   <213> Artificial
<220>
   <223> adapter
<400> 6
   gtaatacgac tcactatagg gcacgcgtgg tcgacggccc gggctggt 48
<210> 7
   <211> 8
   <212> DNA
   <213> Artificial
<220>
   <223> adapter
<400> 7
   accagccc 8
<210> 8
   <211> 22
   <212> DNA
   <213> Artificial
<220>
   <223> adapter primer AP1
<400> 8
   gtaatacgac tcactatagg gc 22
<210> 9
   <211> 34
   <212> DNA
   <213> Artificial
<220>
   <223> UTR2-specific L1 primer
<400> 9
   ggccacactt gacaatccga tataacatgc ctca 34
<210> 10
   <211> 19
   <212> DNA
   <213> Artificial
<220>
   <223> AP2 primer
<400> 10
   actatagggc acgcgtggt 19
<210> 11
   <211> 23
   <212> DNA
   <213> Artificial
<220>
   <223> UTR2-specific L2 primer
<400> 11
   caaatggaaa tgaaatgagg tag 23
<210> 12
   <211> 30
   <212> DNA
   <213> Artificial
<220>
   <223> RbcS-RN
<400> 12
   acccgggccc aggagagcat agaggaagcc 30
<210> 13
   <211> 30
   <212> DNA
   <213> Artificial
<220>
   <223> RbcS-R1
<400> 13
   cggtgaatgg agcgaccatc gtggcttgag 30
<210> 14
   <211> 30
<212> DNA
   <213> Artificial
<220>
   <223> RbcS-R2
<400> 14
   ctgtgaatgg agcaaccatg gccgcttgag 30
<210> 15
   <211> 2132
   <212> DNA
   <213> Artificial
<220>
   <223> codon-optimized human HSA gene
<400> 15
   aagcttgaag acgacatgaa gtgggttact ttcatctctc ttcttttcct tttctcttct    60
   gcttactctg atgctcataa gtctgaagtt gctcatagat tcaaagatct cggagaggag   120
   aacttcaagg ctcttgttct tatcgctttc gctcagtacc ttcagcagtg ccctttcgag   180
   gatcatgtta agctcgttaa cgaggttaca gagttcgcta agacttgcgt tgctgatgag   240
   tcggccgaga actgcgataa gtctcttcat actcttttcg gagataagct ctgcactgtt   300
   gctactctta gagagactta cggagagatg gctgattgct gcgctaagca ggagcctgag   360
   agaaacgagt gcttccttca acataaggat gataacccta accttcctag acttgttaga   420
   cctgaggttg acgtcatgtg cactgctttc catgataacg aggagacttt cctcaagaag   480
   tacctttacg agatcgctag aaggcatcct tacttctacg ctcctgagct tcttttcttc   540
   gctaagagat acaaggctgc tttcactgag tgctgccagg ctgctgataa ggctgcatgc   600
   cttcttccta agctcgatga gcttagagat gagggaaagg cttcttctgc taagcagaga   660
   ctcaagtgcg ctagccttca gaagttcgga gagagagctt tcaaggcttg ggctgttgct   720
   agactttctc agagattccc taaggctgaa ttcgctgaag tttctaagct cgttactgat   780
   cttactaagg ttcacactga gtgctgccat ggtgatcttc ttgagtgcgc tgatgataga   840
   gctgatcttg ctaagtacat ctgcgagaac caggattcta tctcttctaa acttaaggag   900
   tgctgcgaga agcctcttct tgagaagtct cattgcatcg ctgaggttga gaacgatgag   960
   atgcctgctg atcttccttc tcttgctgca gacttcgttg agtctaagga tgtttgcaag  1020
   aactacgctg aggctaagga tgttttcctt ggaatgttcc tttacgagta cgctagaagg  1080
   catcctgatt actctgttgt tcttcttttg agacttgcta agacttacga gactactctc  1140
   gagaagtgct gcgctgctgc tgatcctcat gagtgctacg ctaaggtttt cgatgagttc  1200
   aagccactag tcgaggagcc tcagaacctt atcaagcaga actgcgagct tttcaagcag  1260
   cttggagagt acaagttcca gaacgctctt cttgttagat acactaagaa ggttccacaa  1320
   gtttctactc ctactcttgt tgaggtttca agaaaccttg gaaaagttgg atctaagtgc  1380
   tgcaagcatc ctgaggctaa gagaatgcct tgcgctgagg attacctttc tgttgttctt  1440
   aaccagcttt gcgttcttca tgagaaaaca ccggtgtctg atagagttac taagtgctgc  1500
   actgagtctc ttgttaacag aaggccttgc ttctctgctc ttgaagttga tgaaacgtac 1560
   gttcctaagg agttcaacgc tgagactttc actttccatg ctgatatctg cactctttct 1620
   gagaaggaga gacagatcaa gaagcagact gctcttgttg agcttgttaa gcataagcct 1680
   aaggctacta aggagcaatt gaaggctgtt atggatgatt tcgctgcttt cgttgagaag 1740
   tgctgcaagg ctgatgataa ggagacttgc ttcgctgagg agggaaagaa gctcgttgct 1800
   gcttctcagg ctgctcttgg actttaagag ctcttcgctt tcatctaata atatcttctc 1860
   atttcatttc caataagtct gtttcttttt ttctctttgg atttctgtta cgagactttc 1920
   tatatcggat tgtaaaatgt ctgattttat gaacatgtaa tttctatatt gcttcttcgt 1980
   cttggttact ttccgatggc tattaggttt tcaactctta ttgggataag aagccagtca 2040
   aaataactta acaaaacagg ttagataatg ttagtggtat attgtagaat aagaaaagca 2100
   gcaaacagca gtggtgacct ctagaaggat cc 2132
<210> 16
   <211> 714
   <212> DNA
   <213> Artificial
<220>
   <223> light chain (anti-hevein 1C2) coding region
<220>
   <221> misc_feature
   <222> (1)..(66)
   <223> a sequence coding for mouse signal peptide
<220>
   <221> misc_feature
   <222> (67)..(324)
   <223> light chain anti-hevein 1C2 antigen variable region
<220>
   <221> misc_feature
   <222> (325)..(714)
   <223> kappa light chain constant region
<400> 16
   atggattttc aagtgcagat tttcagcttc ctgctaatca gtgcctcagt cataatatcc 60
   agaggagaaa cgacactcac gcagtctcca tcctccctgt ctgcatctgt aggagacaga 120
   gtcaccatca cttgccgggc aagtcagagc attagcagct atttaaattg gtatcagcag 180
   aaaccaggga aagcccctaa gctcctgatc tatgctgcat ccagtttgca aagtggggtc 240
   ccatcaaggt tcagtggcag tggatctggg acagatttca ctctcaccat cagcagtctg 300
   caacctgaag attttgcaac ttactactgt caacagagtt acagtacccc tcggacgttc 360
   ggccaaggga cacgactgga gattaaacgt cgaactgtgg ctgcaccatc tgtcttcatc 420
   ttcccgccat ctgatgagca gttgaaatct ggaactgcct ctgttgtgtg cctgctgaat 480
   aacttctatc ccagagaggc caaagtacag tggaaggtgg ataacgccct ccaatcgggt 540
   aactcccagg agagtgtcac agagcaggac agcaaggaca gcacctacag cctcagcagc 600
   accctgacgc tgagcaaagc agactacgag aaacacaaag tctacgcctg cgaagtcacc 660
   catcagggcc tgagctcgcc cgtcacaaag agcttcaaca ggggagagtg ttga 714
<210> 17
   <211> 498
   <212> DNA
   <213> Brassica rapa
<220>
   <221> terminator
   <222> (1)..(498)
   <223> rbcs-4 terminator
<400> 17
   ttcgctttca tataataata tcttctcatt tcatttccaa taagtctgtt tctttttttc 60
   tctttggatt tctgttacga gactttctat atcggattgt aaaatgtctg attttatgaa 120
   catgtaattt ctatattgtt tcttcttcgt ggttactact ttcagatggc tattaggttt 180
   tcaatttatt gggataagaa aacagtcaga ataataactt tacaaaactg gttagataag 240
   gttagtggta atattttttt agaataggaa acattactac ctacggaaaa aaattcatac 300
   gaagttaatt agttcatcaa agattcaaat aacaagcaca gttataaaag aaacaagcat 360
   tgtatcattt catcgtcaca ttgacataga tttcaagcat acagtagtag tcatcatttg 420
   atatttgatg tttcacactc atcatatgca gtttctgaga tcgtatacat actattggtg 480
   cattataatt gcaaataa 498
<210> 18
   <211> 1434
   <212> DNA
   <213> Artificial
<220>
   <223> heavy chain (anti-hevein 1C2) coding region
<220>
   <221> misc_feature
   <222> (1)..(57)
   <223> a sequence coding for mouse signal peptide
<220>
   <221> misc_feature
   <222> (58)..(387)
   <223> heavy chain anti-hevein 1C2 antigen variable region
<220>
   <221> misc_feature
   <222> (445)..(990)
   <223> IgG1 heavy chain constant region
<400> 18
   atggaatgga gttggatatt tctctttctc ctgtcaggaa ctgcaggtgt ccactctcag 60
   atcaccttga aggagtctgg tcctacgctg gtgaaaccca cacagaccct cacgctgacc 120
   tgtaacctct ctgggttctc gctcagcacc agcggagtgg gtgtgggctg gatccgtcag 180
   cccccaggaa aggccctgga gtggctcgca ctcatttatt gggatgatga taagcgctac 240
   agtccatctc tgaggaacag actcaccatc accaaggaca catccaaaaa ccaggtggtc 300
   cttacaatga ccaacatgga ccctgtggac acaggcacat atttctgtgc acgcagtgtc 360
   aattatgatg acgtttcggg gacttatcac agccacaact ggttcgaccc ctggggccag 420
   ggaaccctgg tcaccgtctc ctcatccacc aagggcccat cggtcttccc cctggcaccc 480
   tcctccaaga gcacctctgg gggcacagcg gccctgggct gcctggtcaa ggactacttc 540
   cccgaaccgg tgacggtgtc gtggaactca ggcgccctga ccagcggcgt gcacaccttc 600
   ccggctgtcc tacagtcctc aggactctac tccctcagca gcgtggtgac cgtgccctcc 660
   agcagcttgg gcacccagac ctacatctgc aacgtgaatc acaagcccag caacaccaag 720
   gtggacaaga gagttgagcc caaatcttgt gacaaaactc acacatgccc accgtgccca 780
   gcacctgaac tcctgggggg accgtcagtc ttcctcttcc ccccaaaacc caaggacacc 840
   ctcatgatct cccggacccc tgaggtcaca tgcgtggtgg tggacgtgag ccacgaagac 900
   cctgaggtca agttcaactg gtacgtggac ggcgtggagg tgcataatgc caagacaaag 960
   ccgcgggagg agcagtacaa cagcacgtac cgtgtggtca gcgtcctcac cgtcctgcac 1020
   caggactggc tgaatggcaa ggagtacaag tgcaaggtct ccaacaaagc cctcccagcc 1080
   cccatcgaga aaaccatctc caaagccaaa gggcagcccc gagaaccaca ggtgtacacc 1140
   ctgcccccat cccgggagga gatgaccaag aaccaggtca gcctgacctg cctggtcaaa 1200
   ggcttctatc ccagcgacat cgccgtggag tgggagagca atgggcagcc ggagaacaac 1260
   tacaagacca cgcctcccgt gctggactcc gacggctcct tcttcctcta tagcaagctc 1320
   accgtggaca agagcaggtg gcagcagggg aacgtcttct catgctccgt gatgcatgag 1380
   gctctgcaca accactacac gcagaagagc ctctccctgt ccccgggtaa atga 1434
<210> 19
   <211> 558
   <212> DNA
   <213> Artificial
<220>
   <223> signal sequence and TNFR
<220>
   <221> misc_signal
   <222> (1)..(69)
   <223> signal sequence
<220>
   <221> misc_feature
   <222> (70)..(489)
   <223> TNFR sequence
<400> 19
   atggcgcccg tcgccgtctg ggccgcgctg gccgtcggac tggagctctg ggctgcggcg 60
   cacgccttgc ccgcccaggt ggcatttaca ccctacgccc cggagcccgg gagcacatgc 120
   cggctcagag aatactatga ccagacagct cagatgtgct gcagcaaatg ctcgccgggc 180
   caacatgcaa aagtcttctg taccaagacc tcggacaccg tgtgtgactc ctgtgaggac 240
   agcacataca cccagctctg gaactgggtt cccgagtgct tgagctgtgg ctcccgctgt 300
   agctctgacc aggtggaaac tcaagcctgc actcgggaac agaaccgcat ctgcacctgc 360
   aggcccggct ggtactgcgc gctgagcaag caggaggggt gccggctgtg cgcgccgctg 420
   cgcaagtgcc gcccgggctt cggcgtggcc agaccaggaa ctgaaacatc agacgtggtg 480
   tgcaagccct gtgccccggg gacgttctcc aacacgactt catccacgga tatttgcagg 540
   ccccaccaga tctgtgag 558
<210> 20
   <211> 576
   <212> DNA
   <213> Artificial
<220>
   <223> Arabidopsis VSP1 (vegetative storage protein-1) and a part of rbcs-4 terminator
<220>
   <221> misc_feature
   <222> (1)..(226)
   <223> Arabidopsis VSP1
<220>
   <221> misc_feature
   <222> (227)..(576)
   <223> a part of rbcs-4 terminator
<400> 20
   ttaagcatct atcttcatgg cattgtcccc ttgtatccat ttcatatcta tgtcgtttcg 60
   tttatctttg tagccgtttt ggcaccactg cttaaataaa atgccaatcc tatcataact 120
   caataagtac aacgacttcg tactaaattt tgtttttcgt taaagggatc attaatcaag 180
   tttccatgaa atgatgaaca tgtaatttct atattgtttc ttcttcgtgg ttactacttt 240
   cagatggcta ttaggttttc aatttattgg gataagaaaa cagtcagaat aataacttta 300
   caaaactggt tagataaggt tagtggtaat atttttttag aataggaaac attactacct 360
   acggaaaaaa attcatacga agttaattag ttcatcaaag attcaaataa caagcacagt 420
   tataaaagaa acaagcattg tatcatttca tcgtcacatt gacatagatt tcaagcatac 480
   agtagtagtc atcatttgat atttgatgtt tcacactcat catatgcagt ttctgagatc 540
   gtatacatac tattggtgca ttataattgc aaataa 576
<210> 21
   <211> 1149
   <212> DNA
   <213> Brassica rapa
<220>
   <221> misc_feature
   <222> (1)..(1149)
   <223> Rbcs-1
<400> 21
   ctggtaattc tgttttaata acactgtcat gaaaactgaa ttcataggtt tctccaggcc     60
   gtagatagtg gaagcctaga tcgtcgtcgt tagatataca attaatcttc aagatattgt    120
   ttcgaccaag agagttctta aagtgcacgg agtttttttc attccacttt gcgttactca    180
   aacctatgca caatccaata gtgagaagaa aacacgagag acgattcatt gtgttgatta    240
   tttgtaatgg ccttcttcta tagttctttt aaatttagaa gtaatccgta gataaccaag    300
   agggaagatg tgtaatatat atatatatag tggcgttaca cggataagca ctcatttttt    360
   ctctattttt aaacatcttt gttttgacta atattaagaa aacgttgatc gcttttacta    420
   tttttcgtgt ccttatcccg tgttgctgtt ttgcatctat ttaaagagta gatgctatag    480
   tttttttacg gcttagacta gatgcttttg gtaatttgtt tcagtgttca gctttgaccc    540
   cctttttttg gtgtaaatgt taaaattcag ccttcagctt tgacctttgt gatattatat    600
   gaatgactgt gtttatgtaa aatttattga tttataaaac tcttaagaaa aactatatta    660
   ataaaaaata gaattgttac tcttcttcgt ttgagtatga acataatcat caatagtgct    720
   ctcatcactc ataagttata actaatgacg acattacatg tctgattcaa gtaatttaat    780
   tttcttgtag tacaggtcca cgaagattta aatgtaggtc atgcgtctca tttcttttct    840
   gccaaaagga aggagccaag agaatcggct caagtggaca ctagtaacca tacacattca    900
   ctcattacct tccaagaaaa gataagataa gaaaattttc tgccacgtgg ccttatcata    960
   gtggtctgta tcgataaggg tgtcaacacc tttccttaat cctgtggcag gtaacgacgt   1020
   tatcatcaga catgaatccc gcactctttg atctaagggc tttatgcctc atgccgncct   1080
   cactatatat agatgaccaa aggaatagac aaacaagtaa gtaagagaaa agagaaaaaa   1140
   gaagtagta                                                           1149
<210> 22
   <211> 877
   <212> DNA
   <213> Brassica rapa
<220>
   <221> misc_feature
   <222> (1)..(877)
   <223> Rbcs-3
<400> 22
   ctggtgattc tattttaata gattaaatat tattctttag aagacgaagc atgagatcga     60
   tgacaacgca atgctcattt catctggttt ttctgtcctt gcctcaattg gttactgtta    120
   cagatgttaa taaatgagta attaccaata ggatacccta agtttttcca aactttcaaa    180
   cccaaaactt actctatttg acttaagagc atttctaaga gcaatgttac ttttagagca    240
   tttctaagag caatgttact tttaaagttt agtgaacttt aaatttgagg tttaagagca    300
   tgtccaatgg ttagaagaca gacttacagt ttaactaccg tgttcagtta ccaatcgggc    360
   ccactatata atattgtttc ccttcttcgt ttatcgacgt tagcattaac agtgtactcc    420
   ttgctcacaa ctaagtagct cataacttgt aatagttata atactaaatg tgatcttatt 480
   aattaactga atcaaactaa gccaatagtt aggagttcca ctaacattaa agagtaggcc 540
   atgtgtttga tttgtcgtct cccaatagga aggaaccaaa agcatcggct caagtggagg 600
   ccagcagtaa atcagtaacc atatatacac acattcatac attttaatgc aagataagat 660
   aatagcattt ctgccacgtg gccttaacat agtggtcagt atcgataagg gtctcaacac 720
   ctttctttaa tcatgtggca gttaacgacg ttatcatgaa atctggaccc tctggttatt 780
   agggcctttt cctcttgcgg ttctcgttat atatagataa ccaaagcgat agacaaacaa 840
   gtaagttaag agaaaagaag aagaagaaga agtagta 877
<210> 23
   <211> 904
   <212> DNA
   <213> Brassica rapa
<220>
   <221> misc_feature
   <222> (1)..(904)
   <223> Rbcs-5
<400> 23
   gtcgacaaaa ctcttctttt atgtcgcagg atggtccgaa tgagacaata acaccttcca 60
   tccttctgat gaggtcacat attcaatcat gtcagttaaa gtcacatatc catgcgatgc 120
   gaatgcagct tgcttcgtct caatgtatta aatacgaatc agactgtatt gttcctactt 180
   gatgacagag tcaacatact tttcccaaag catgggcgtg acgtcgtcac gtagatcgca 240
   ttattgtctt ttatcatata atgaaccggg ctacctttat ctttgggttt ttgaatttgt 300
   tgggctcttt atagaatcgg atcgggcatt gatgagagtg tgaagtccac ctccaacaaa 360
   aactcttgaa ctataactaa tgatgtcctt gtattattag tgataacttt tctaagtaat 420
   gtattgtgga aaattacatg tttgattcaa atcattttct tattaataat taaatattga 480
   ttgagaacta actaaccgaa tcgatgtaca tgccaaactg aatcttacac aattgaacat 540
   aagttcaaac cattaaaagt aggtcaggtt ctgattcttt tttcaattgg aaggagtcaa 600
   aagcatgact caagtggaca ccagtaacca tacacattca ctcattccct cacaagaaac 660
   gataagataa tggaattttc tgccacgtgg ccttatcata gtggtctgta ttgataaggg 720
   tgtcaacacc tttccttaat cctgtggcag gcaacgacgt tatcatgaat cttggaccca 780
   tttattacta gggctttttg tctcttgccg ttctcactat ataaagatga ccaaagcaat 840
   agacaagcaa gtaaaagaaa ggagaaaaag aagaagaaga agaagagaag aactagtaca 900
   cgta 904
<210> 24
   <211> 12
   <212> DNA
   <213> Artificial
<220>
   <223> KDEL signal
<400> 24
   aaagacgagc tg 12
<210> 25
   <211> 25
   <212> DNA
   <213> Artificial
<220>
   <223> RbcS-2-3' UTR-reverse
<400> 25
   tataacatgc ctcagaaaca aaaag 25
<210> 26
   <211> 28
   <212> DNA
   <213> Artificial
<220>
   <223> RbcS-3-3' UTR-reverse
<400> 26
   cgatatagaa tgtctgagaa acagaaaa 28
<210> 27
   <211> 27
   <212> DNA
   <213> Artificial
<220>
   <223> RbcS-4-3' UTR-reverse
<400> 27
   cgatatagaa agtctcgtaa cagaaat 27
<210> 28
   <211> 24
   <212> DNA
   <213> Artificial
<220>
   <223> RbcS-5-3' UTR-reverse
<400> 28
   ctcagaaaca aaaattcaaa agca 24
<210> 29
   <211> 21
   <212> DNA
   <213> Artificial
<220>
   <223> RbcS-2-3' UTR-forward
<400> 29
   ggtgcttaat tcgcgttgta a 21
<210> 30
   <211> 26
   <212> DNA
   <213> Artificial
<220>
   <223> RbcS-3-3' UTR-forward
<400> 30
   tgcttaattt gctatgacat tcacat 26
<210> 31
   <211> 23
   <212> DNA
   <213> Artificial
<220>
   <223> RbcS-4-3' UTR-forward
<400> 31
   cggtgcttaa ttcgctttca tat 23

<210> 32
   <211> 23
   <212> DNA
   <213> Artificial
<220>
   <223> RbcS-5-3' UTR-forward
<400> 32
   ggcgcttaat tttgttgtct aaa 23

## Description

### Field of the Invention

The present invention relates to transgenic plants. More specifically, it relates to transgene expression at a specific stage of plant development. Even more specifically, the invention relates to Rubisco promoters of *Brassica,* and DNA constructs or expression cassettes comprising at least one of said promoters for the transformation of homologous or heterologous plants for efficient production of gene products, particularly, for contained use.

### Background of the Invention

Assimilation and conversion of atmospheric carbon dioxide via the reaction with ribulose-1,5-bisphosphate into phosphoglycerate strictly depends on the activity of Rubisco enzyme (Spreitzer, R.J., Arch. Biochem. Biophys. 414(2), 141-9, 2003; Spreitzer, R.J., et al., Annu. Rev. Plant Biol., 53, 449-75, 2002). Structurally, the Rubisco enzyme consists of eight small subunits (SSU) and eight large subunits (LSU). The SSU proteins are encoded by several genes, which are located in the nuclear genome of plants, while LSU genes are found in plastid genomes. The number of Rubisco SSU genes in different plants varies from four copies up to fifteen copies or more in some polyploid genomes. At least four copies of Rubisco SSU genes are known to be present in *Arabidobsis thaliana* and twelve or even more copies per polyploid genome are known in wheat (Sasanuma, T., Mol. Genet. Genomics. 265 (1),161-171,2001; Sasanuma, T. and Miyashita, N.T., Genes Genet. Syst.,73(5), 297-309, 1998).

The Rubisco enzyme is one of the most ubiquitous enzymes present in plants. Promoters of the genes encoding the Rubisco enzyme have without specifying any of their particular characteristics been suggested as strong promoters useful for the preparation of DNA constructs for transforming plants (US 5,994,628, US 6,664,109 corresponding to US 2002/0170096, US 2003/0097678).

The structure, evolution and regulation of *RbcS* genes in higher plants has been described by Dean, C., et al (Annu. Rev. Plant Physiol. Plant Mol. Biol., 40, 415-439, 1989) and based on the structure and function of the *RbcS* genes, Dean et al. suggested that the nuclear genes may be grouped in multigenic families. The gene structures of these families have been extensively studied in other plants, such as *Arabidopsis*, tomato, *Chrysanthemum, Brassica napus* and soybean. In tomato there are five Rubisco SSU (*rbcS*) genes, which are located in 3 chromosomal loci, one in chromosome 3, and the other four in chromosome 2 (Sugita, M., et al., Mol. Gen. Genetic., 209 (2), 247-256,1987). Moreover, three of the genes are known to be organized in tandem array within a 10 kb region. The situation is quite similar in the *Arabidopsis thaliana rbcS* gene family (Krebbers, E., et al., Plant Mol. Biol. 11, 745-759, 988; Niwa, Y., et al., NA Res., 4(5), 341-343, 1997; Dedonder, A., et al., Plant Physiol., 101, 3, 801-808, 1993), and in wheat (Galili, S., et al., Mol. Gen. Genet., 263 (4), 674-680, 2000).

Outchkourov, N.S. et al. (Plant, 216 (6), 1003-1012, 2002) cloned an abundantly transcribed *rbcS1* of the Rubisco small-subunit gene family of a *Chrysanthemum* species (*Chrysantemum morifolium* Ramat.) and demonstrated that tobacco plants transformed with a gene cassette containing an *UidA* gene expressing GUS under the control of a *rbeS1*-promoter provided up to 10% GUS of total soluble proteins in the leaves. Even if said Rubisco promoter from *Chrysanthemum* gives high protein expression levels in tobacco leaves, high expression in other developmental stages has not been demonstrated.

US 6,664,109 corresponding to US Patent application 2002/0170096 discloses the use of the promoter region of a soybean SSU gene in a transformation system with Ri- or Ti-plasmids.

In *Brassica napus* coding sequences, 5'- and 3'-regulatory regions of three *rbcS* genes (accession numbers X75334, X55937, X61097) have been cloned and sequenced (Beck, I., et al., Bot. Acta, 108, 327-333, 1995; Fiebig, C. and Link, G., Curr. Genet., 21(2), 161-8, 1992; Fiebig, C., et al., Bot. Acta, 103, 258-265, 1990). According to the data obtained with Southern analysis *Brassica napus* is believed to contain no more than three *rbcS* genes (Nantel, A. M., et al., Plant Mol. Biol., 16(6), 955-966, 1991). cDNA sequences obtained from mRNA of *Brassica napus rbcS* genes have been described by Baszczynski, et al. (Nucleic Acids Res. 16(10), 4732, 1988) One of the published cDNAs has been provided with the accession number X07367. Beck, et al., Bot. Acta, 108, 327-333, 1995 has described transient expression activity of *RbcS* regions from *Brassica napus* in mesophyll protoplasts from tobacco.

The *rbcS* gene family of other *Brassica* species is much less studied than the *Brassica napus rbcS* gene family. In fact, the data available on the gene structure and activity does not enable the identification of differently expressing members of *Brassica rbcS* gene family in different plant tissues or during different spatiotemporal stages or under various environmental conditions.

Because leaves are abundantly produced and the leaf size is big, expression and production of gene products in plants are often demonstrated in leaves and the gene products are harvested from them. In order to enable production of transgenic expression products in developing sprouts it is of great importance to have promoters expressing strongly, for example, during selected stages of cotyledon development. Plant seeds and cotyledons are particularly advantageous for production, because at early cotyledon development, nutritional sources from seeds, including amino acids and oils are abundantly available as raw material for *de novo* synthesis and the recovery of the expressed gene products from the substrate solution is easier and more efficient than from harvested leaves.

An important aspect when designing transgenic plants is how to obtain significant levels of transgene expression in the desired plant tissues or during desired plant development phases. The role of promoters is essentially important in this aspect and therefore, new promoters with different properties and expression profiles are needed.

The provision of a high level of transgene expression is not the sole aspect to take in consideration, when producing gene products in transgenic plants. Especially, when producing foreign or heterologous gene products in contained conditions, the provision of strong expression in a selected, specific, spatiotemporal manner, which allows production of gene products in selected plant tissues during a selected, preferably quite short time period, is of particular importance. Promoters, which are active during seed germination or the development of the cotyledon are needed for the production of transgene products in contained conditions in suitable facilities. New promoters for different new applications are therefore needed.

The objective of the present invention is to obtain a spatiotemporally targeted high expression level of the desired gene product or protein. Therefore, new promoters not only expressing at different developmental stages, but particularly such that provide a high protein level at a specific development stage and/or in a specific organ of a plant are provided.

### Summary of the Invention

The present invention is related to a family of novel spatiotemporally active Rubisco promoters including SEQ ID NO:1 or SEQ ID NO:3, which are obtainable from light grown *Brassica* seedlings and to transgene expression in specific plant organs or at specific stages of plant development. The promoters are useful for designing DNA constructs or cassettes, comprising at least one of said promoter sequences functionally fused in frame with genes encoding the desired gene products. Seeds from transformed homologous or heterologous zero generation plants and from subsequent generations of the transformed plants are collected and used for efficient production of gene products, particularly in contained conditions.

The promoters are used to design expression cassettes, which comprise at least one Rubisco promoter selected from the family of Rubisco promoters including the nucleotide sequences SEQ ID NO:1 or SEQ ID NO:3, which promoter sequences are operably linked to a heterologous or homologous nucleic acid sequence encoding a desired protein or gene product.

The nucleic acid sequences are exemplified by reporter genes, which include naturally occuring isolated genes or synthetic or semisynthetic nucleic acid sequences partially designed from isolated naturally occuring genes. The reporter genes encode, for example, GUS, human serum albumin (HSA), antibodies and medically active proteins.

The DNA constructs or cassettes are used for transforming host plants, which are exemplified by *Brassica* and *Camelina* species. This transformed so called zero generation plants comprise one or more of Rubisco promoter containing DNA cassettes. Seeds of said zero generation transformed plants may be used for providing further generations of plants, but naturally the seed can be directly used for production of the desired gene products from seedlings of said seeds during seed germination and cotyledon development.

Therefore, the present invention is related to transformed plants, subsequent generations of said transformed plants as well as to seed or seedlings, carrying at least one DNA construct or cassette comprising a Rubisco promoter sequence SEQ ID NO:1 or SEQ ID NO:3 operably linked to a nucleic acid sequence encoding a heterologous protein or gene product.

### Brief Description of the Drawings

Figure 1 illustrates relative quantities of different 3'UTR-type *rbcS*-genes in germinating seeds of *Brassica rapa.*
Figures 2A, 2B, 2C, 3, and 4 demonstrate DNA sequence alignments with the Global DNA alignment program. Reference molecule: rbcS-300nt. Region 1 to 300. Sequences 5. Scoring matrix: Linear [Mismatch 2, OpenGAp 4m ExtGap 1]. Sequence view: Similarity Format, Color areas of high matches at the same base position.

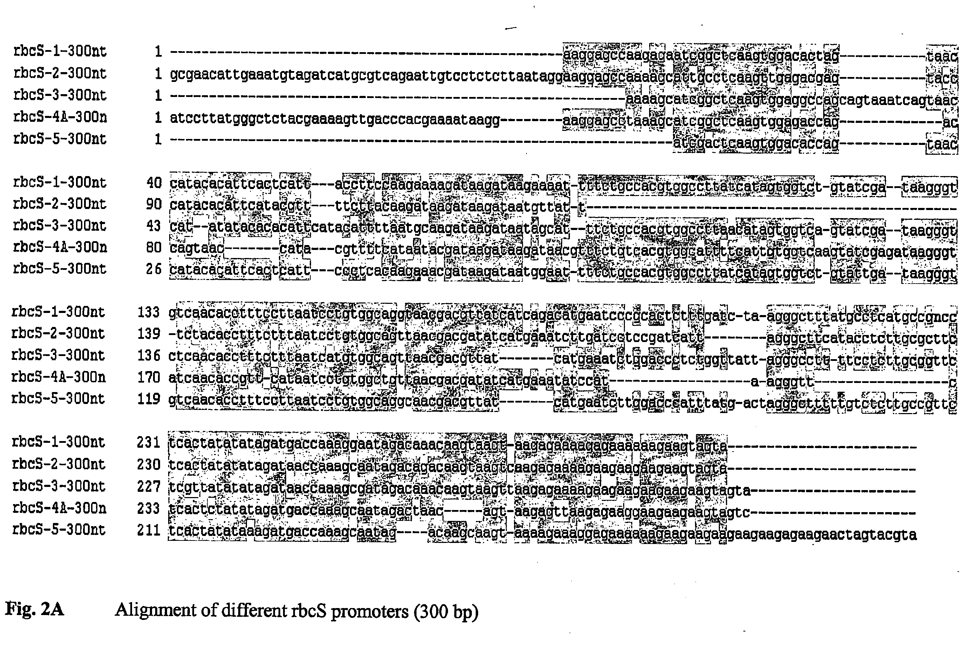

Figure 2A illustrates alignments of cloned *Brassica rapa rbcS* promoters (300 bp)

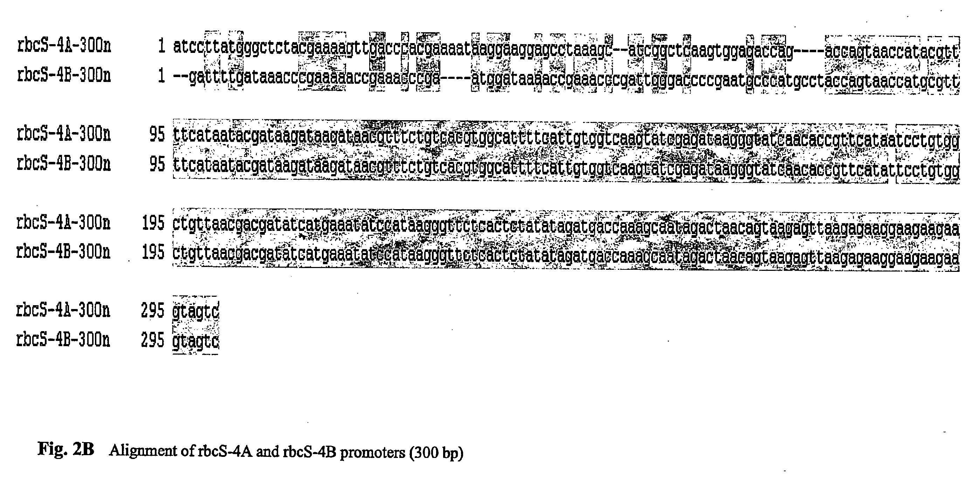

Figure 2B illustrates alignments of cloned *Brassica rapa rbcS-4A* (SEQ ID NO:1) and *rbcS-4B* (SEQ ID NO:2) promoters (300 bp), in which the horizontal boxes are homologous regions).

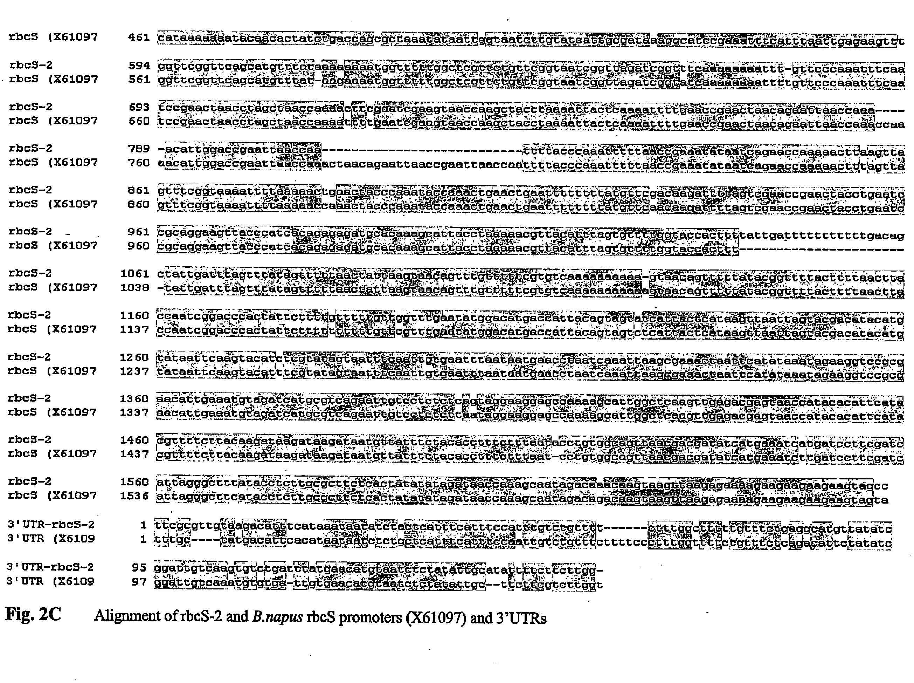

Figure 2C illustrates alignments of promoters (upper part) and 3'UTRs (lower part ) *Brassica rapa rbcS* -2 (SEQ ID NO:3) and *B napus rbcS* (X61097).

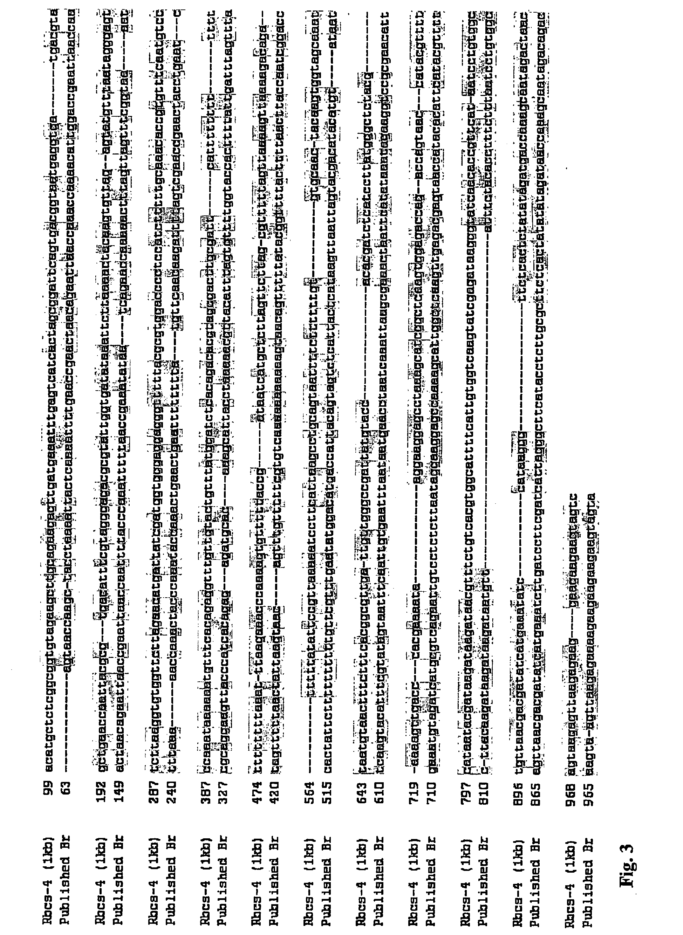

Figure 3 illustrates alignments of 1 kb sequences of *Rbcs-4A* promoter (SEQ ID NO:1)(upper line) and *Brassica napus* Rubisco promoter published with access number X61097 (lower line) The sequences show 52% dissimilarity.

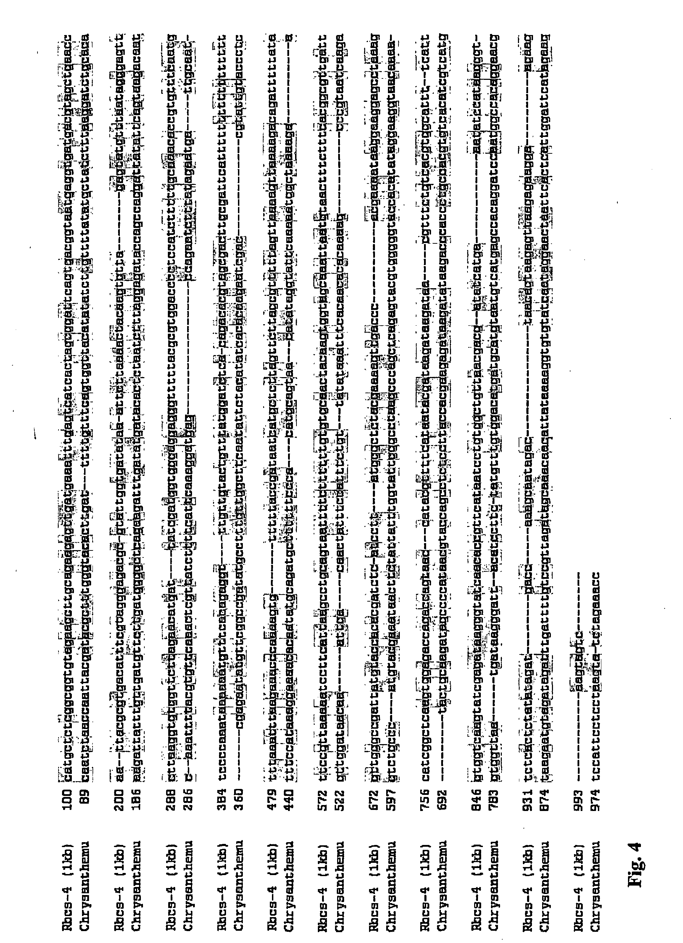

Figure 4 illustrates alignment of 1kb sequences of *rbcS-4A* promoter (SEQ ID NO:1) (upper line) and *Chrysanthemum rbcS1* promoter published with access number AY163904. The sequences show 57% dissimilarity.

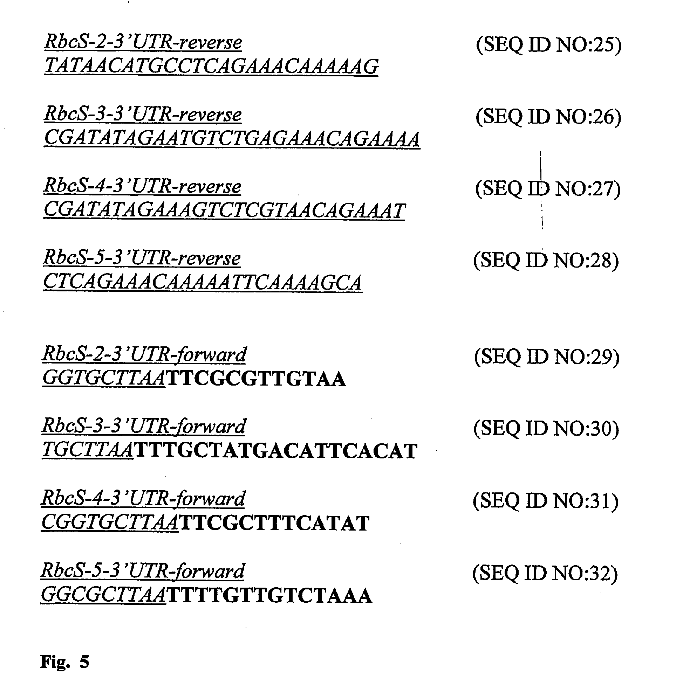

Figure 5 illustrates sequences of the forward and reverse primers specific for unique parts of different rubisco 3'UTR types. The primers show that different 3'UTR species could be differentiated with real-time PCR. Forward primers are divided in two parts (underlined and italics): the left part of each primer corresponds to the last few nucleotides of relevant rubisco coding regions, and the right part corresponds to specific 3'UTR sequence. All the reverse primers anneal to specific 3'UTR regions. All the amplicon sizes vary in 80-100 nt range.

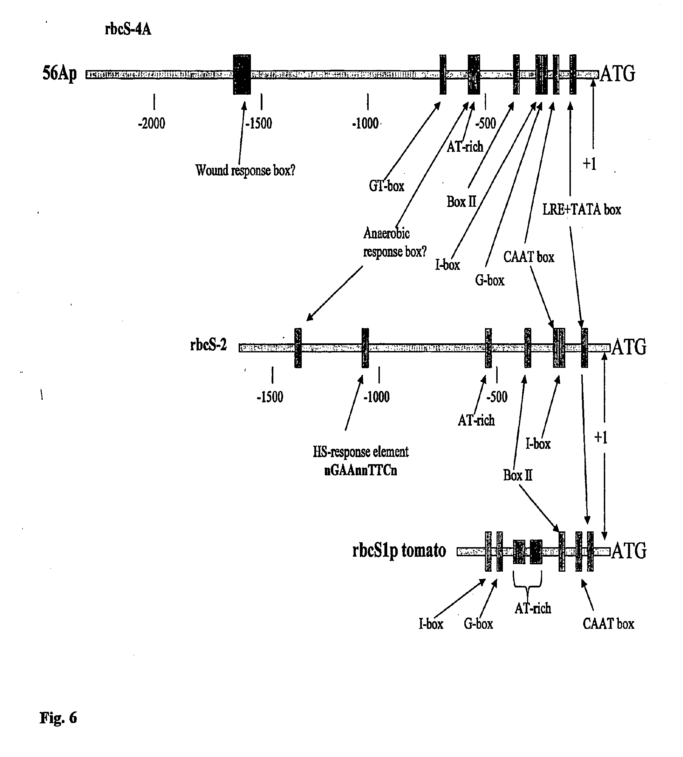

Figure 6 is a scheme of consensus regulatory elements found in *rbcS-2* (SEQ ID NO:3) and *rbcS-4A* (SEQ ID NO:1) promoters.

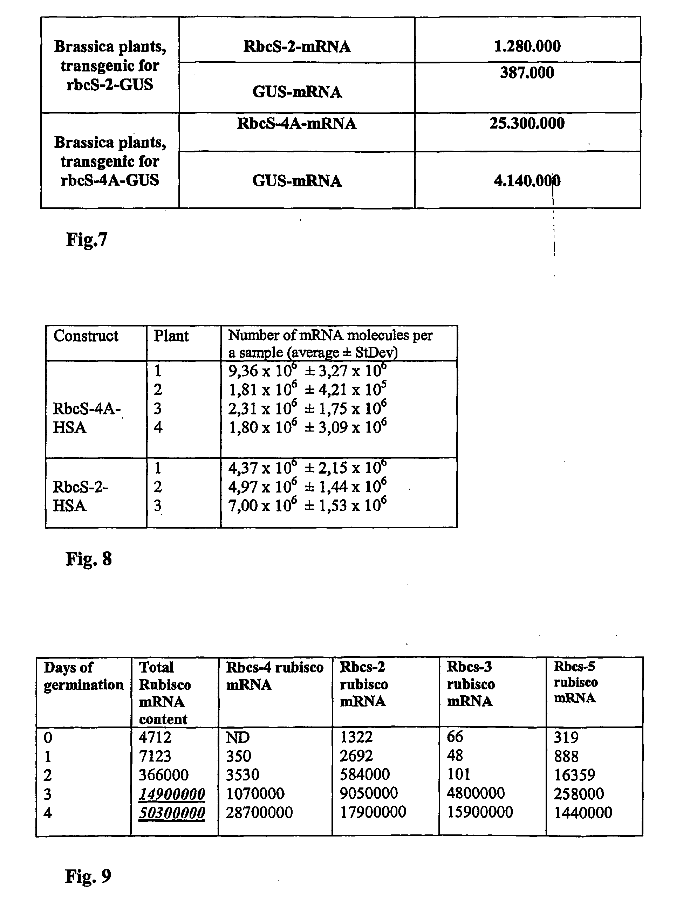

Figure 7 is a Table demonstrating the amount of mRNA from *rbcS-2, rbcS-4* and *UidA* (GUS) using real-timePCR in transgenic *Brassica* plants transformed with *rbcS-2-GUS* and *rbcS4A-*GUS (Rubisco promoters sequences (SEQ ID NO:3 and SEQ ID NO:1, respectively)
Figure 8 illustrates the amount of HSA mRNA molecules in RNA samples of transgenic tobacco plants (real-time PCR data).
Figure 9 illustrates expression levels of different Rubisco promoters in *Brassica napus* seeds, germinated for various times. Data represents numbers of molecules per a sample (100 ng of total RNA).

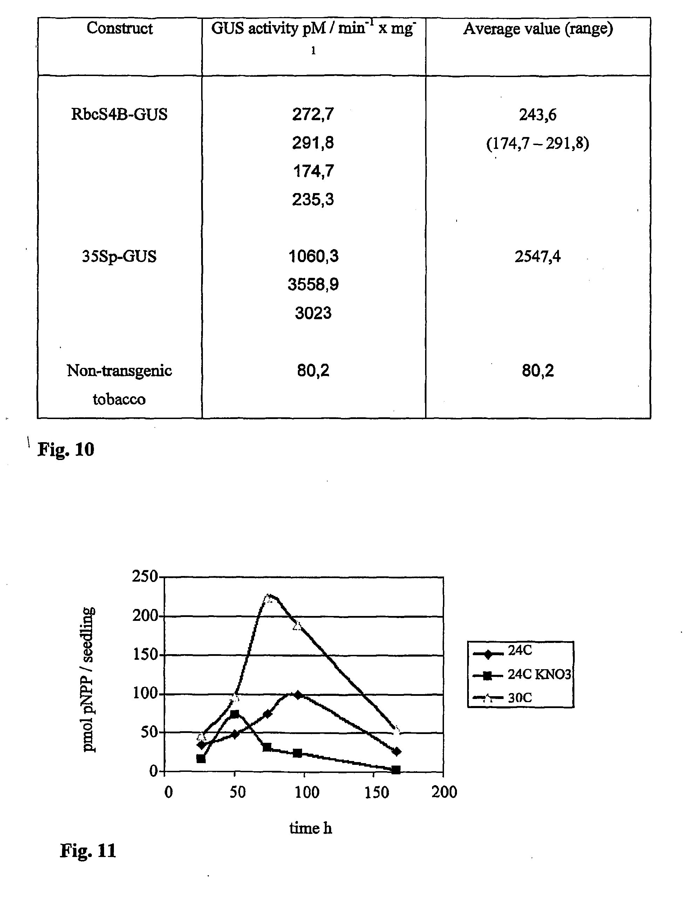

Figure 10 illustrates the GUS expression in transgenic tobacco plants carrying *rbcS-4B UidA* gene comprising construct.
Figure 11 illustrates GUS expression under Rubisco promoter *rbcS*-4A during germination of *Brassica* seeds in constant light at 24 °C or 30 °C.

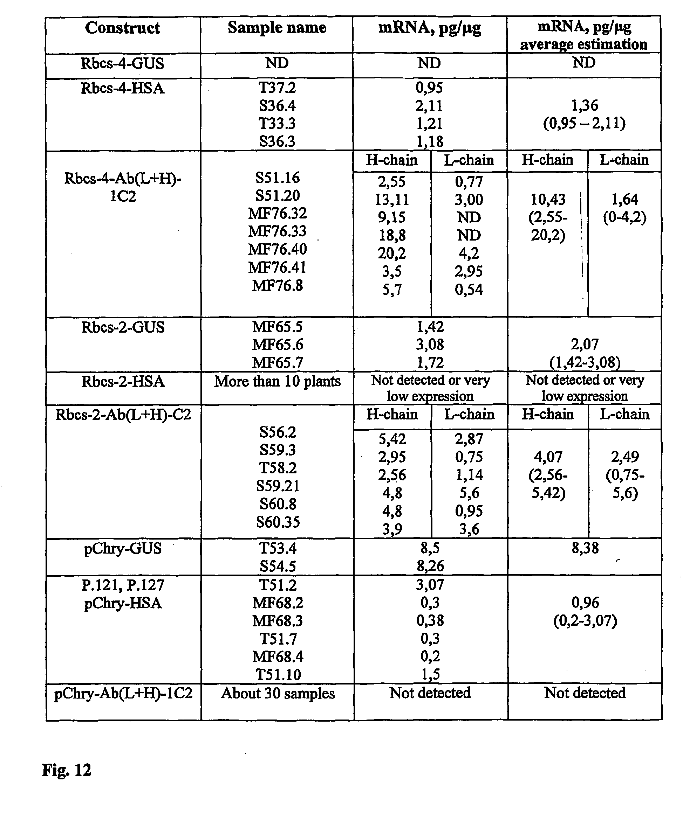

Figure 12 illustrates HSA mRNA content in transgenic *Camelina* plants (Northern analysis).

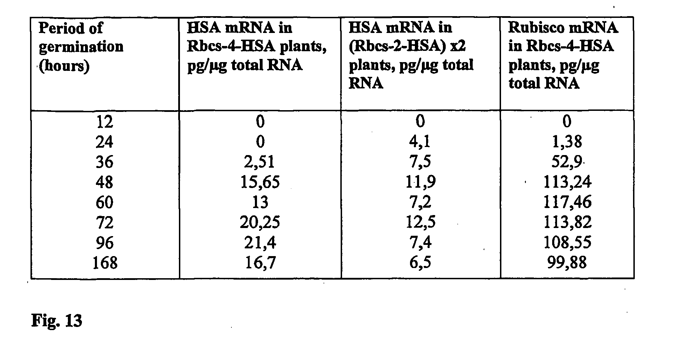

Figure 13 illustrates HSA mRNA content in germinating seeds of transgenic *Brassica napus* plants at various times.

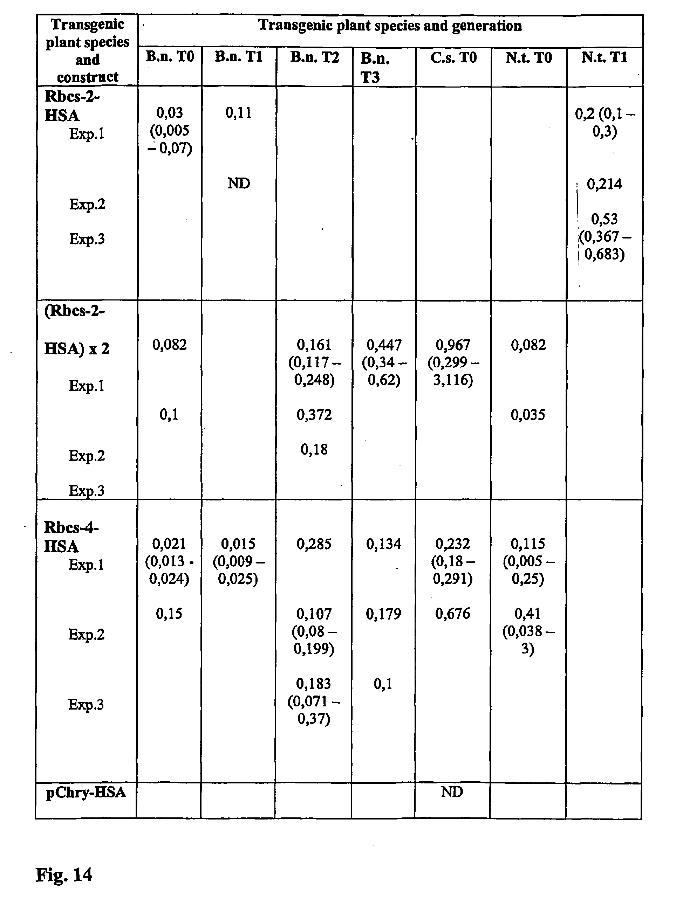

Figure 14 illustrates the amount of HSA protein in transgenic Brassica, Camelina and tobacco plants calculated as % TSP (total soluble protein). Averages with minimum and maximum values are presented.

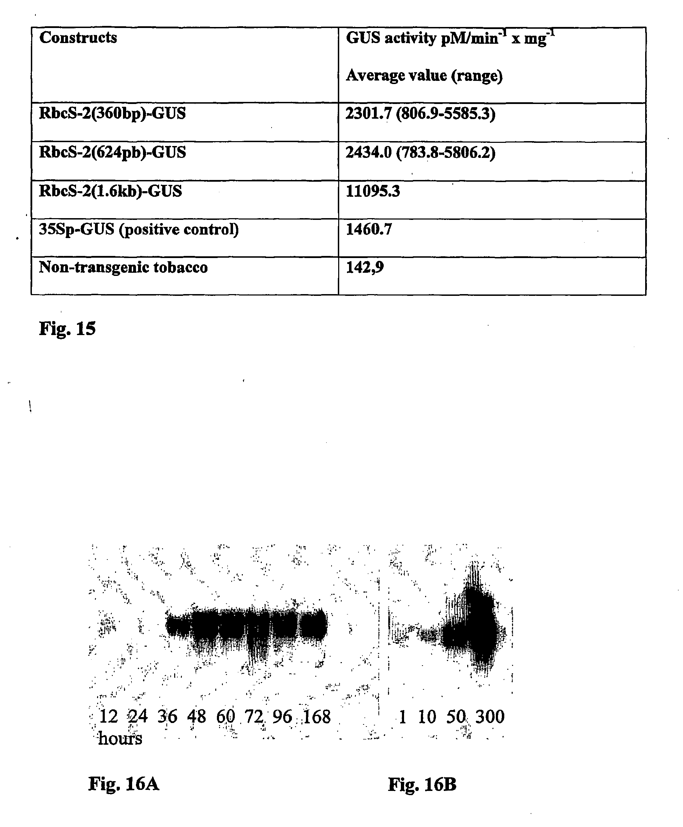

Figure 15 illustrates the GUS activity in transgenic tobacco plants transformed with constructs comprising GUS as the reporter gene and either full length *rbcS-2* (SEQ ID NO:3) promoter (1.6 kb) or a truncated version of the promoter. 35S-promoter is used as a positive control.
Figure 16 illustrates expression of Rubisco promoters in germinating *Brassica* seedlings as revealed by Northern blot analysis.
Figure 16A. is a Northern blot showing the synthesis of Rubisco SSU mRNA in *Brassica* seedlings after sprouting in an airlift tank for 12 to 168 hours.
Figure 16B shows unlabelled Rubisco RNA produced by *in vitro* transcription when loaded on the same filter as the control. The amount of control RNA is indicated in pg.

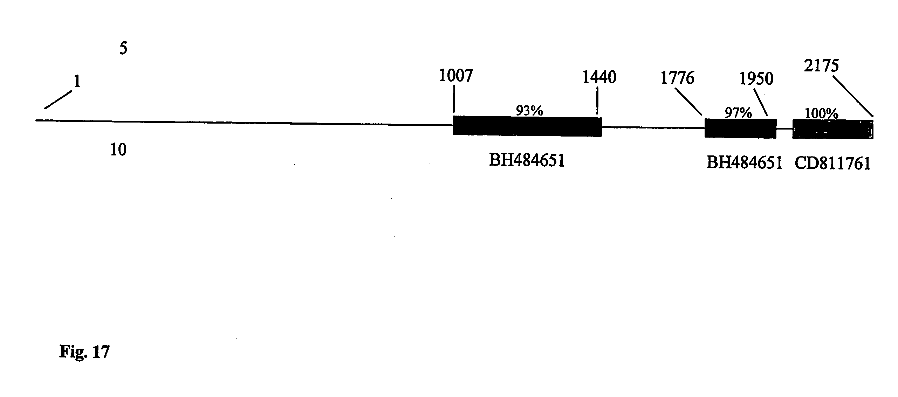

Figure 17 illustrates alignment of the sequence of RbcS-4A (SEQ ID NO:1) promoter (upper line) and *Brassica* sequence published in the *Brassica* genome project with the accession number BH 484651.

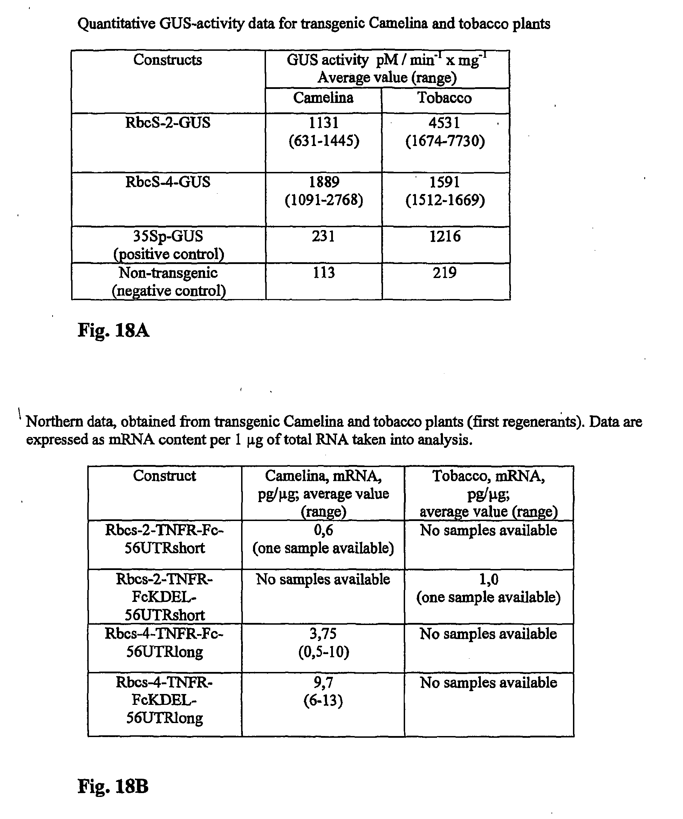

Figure 18 illustrates a quantitative Northern data for transgenic *Camelina* and tobacco plants carrying TNFR-constructs. In the Figure Rbcs-2-TNFR-Fc-56UTR short contains rbcs-2 promoter, TNFR part (489 nt), linked to the part of IgG1 heavy chain constant region (C_{H}2+C_{H}3 domains), and terminator from natural rbcs-4 gene (0,5 kb length), is the same as in previous construct, but there is also KDEL signal (12 nt) after Fc region (just before STOP codon). Rbcs-2-TNFR-Fc-56UTRlong contains rbcs-4A promoter, TNFR part (489 nt), linked to the part of IgG1 heavy chain constant region (C_{H}2+C_{H}3 domains), and terminator from natural rbcs-4 gene (2 kb length). Rbcs-4-TNFR-FcKDEL-56UTRlong is the same as previous construct, but there is also KDEL signal (12 nt) after Fc region (just before STOP codon).

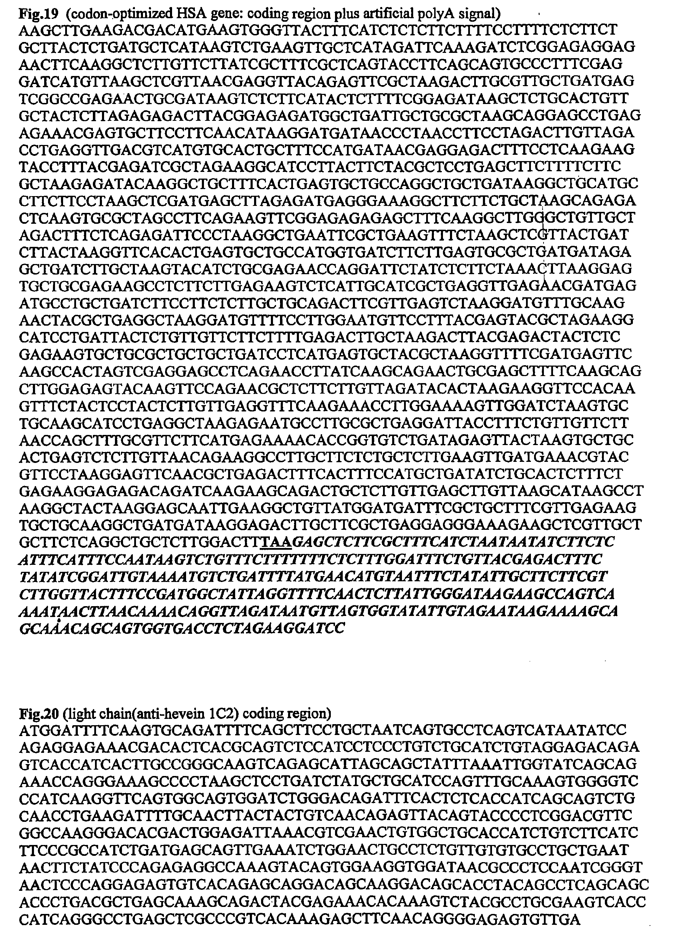

Figure 19 depicts an artificial HSA gene (SEQ ID NO:15). The sequence of an isolated natural human gene (cDNA, i.e. only exons and no introns) has been codon-optimized
Figure 20 depicts an artificial or semisynthetical light chain(anti-hevein 1C2) coding region (SEQ ID NO:16). The sequence consists of 3 parts:
   66 nt length sequence coding for mouse signal peptide (22 amino acids). The sequence has a 100% similarity with the partial sequence having the accession number AF078548;
   324 nt length light chain anti hevein 1C2 antigen variable region is isolated from a phage display library (obtained from VTT). Accession number AB095291 (Genbank) has a 100% similarity to the 16-305 nt region of the variable region in Figure 20 (SEQ ID NO:16);
   324 nt length of kappa light chain constant region, which has a 100% similarity with the sequence having accession number BC063599.

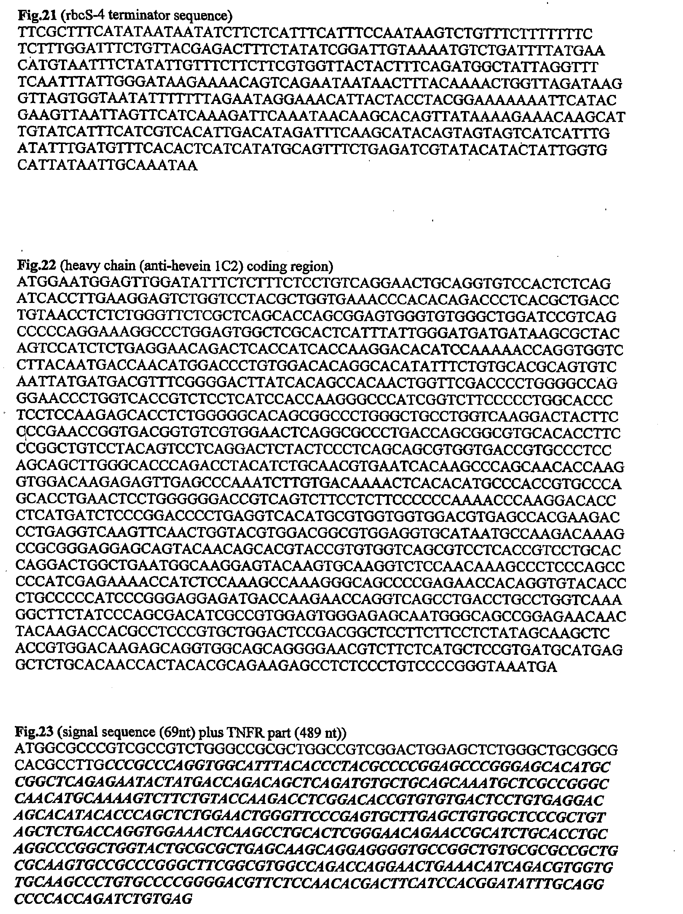

Figure 21 depicts an artifical *rbcS*-4 terminator sequence (SEQ ID NO:17). Originally this sequence was cloned from the genome of *Brassica rapa* by Genome Walking techniques. Similar sequences are published in the *Brassica* Genome Project. Accession number BH691838 has a partial similarity of 88%.
Figure 22 depicts an artificial heavy chain (anti-hevein 1C2) coding region (SEQ ID NO:18). The sequence consists of 3 parts:
   57 nt length sequence coding for mouse signal peptide (17 amino acids). A part of the sequence having accession number X67210 has a 100% similarity;
   387 nt length heavy chain anti hevein 1C2 antigen variable region made by phage display techniques (obtained from VTT). Accession number AB067222 (Genbank) has 95% similarity to the 1-295 nt region of the variable region shown in the Figure 22;
   990 nt length of IgG1 heavy chain constant region. A sequence having the accession number BC024289 has a 99% similarity. The minor differences are possible because of allelic differences.
Figure 23 depicts an artificial signal sequence (69nt) plus TNFR part (489 nt). A sequence having the accession number NM_001066 has a 100% similarity with the sequence, which is also disclosed in US 5,605,690. Similar sequences are obtainable from other TNFR sequence.

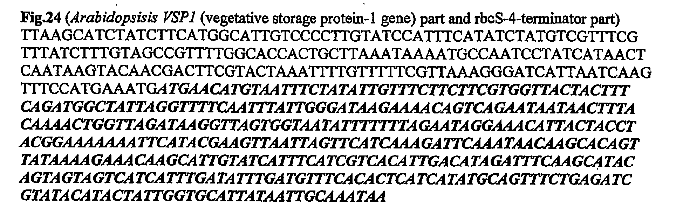

Figure 24 depicts an artificial part of the *Arabidopsisis* VSP1 (vegetative storage protein-1 gene) -3' UTR and a part of the rbcS-4-terminator) (Genbank accession number NM_122387). *RbcS*-*4*-terminator part herein is the full length *rbcS*-*4*-terminator shown in Figure 21, starting from behind the cleavage site and until nt 350.

It is to be noted that the reporter genes disclosed in the present Figures are not part of this invention. They are only used to exemplify the feasibility of the Rubisco promoters of the present invention.

### Detailed Description of the Invention

The terms used in the present invention have the meaning they usually have in the fields of recombinant DNA technology and transgene expression in plants. Some terms in the present invention are, however, used in a broader or somewhat different manner. Therefore, some of the terms are defined in more detail below.

### Definitions

The term **"reporter gene"** means genes coding for homologous or heterologous proteins or other metabolic gene products, which can be demonstrated either easily as the GUS enzyme or by applicable known methods of determination, such as spectrometric methods, immunoassays, etc. The reporter gene is a"structural gene", which may code for any selected or desired protein or gene product. The reporter genes are exemplified the by the *uidA* gene, which encodes the GUS protein and the gene encoding human serum albumin (HSA) SEQ ID NO:15. Said genes are particularly useful as model genes in research and for the development of industrially useful DNA constructs or cassettes, because they encode easily detectable marker proteins.

The term **"homologous promoter"** means a promoter that is endogenous to the host plant species. In other words, it is native to or present in the same plant species from which the host plant originates. In other words it is native to host plant species.

The term **"heterologous promoter"** means that the promoter is exogenous to the host plant species. The promoter is not present in an untransformed plant species. In other words, it is not native to the host plant and the host plant must be transformed with a construct carrying said promoter.

The term **"homologous system"** means a DNA construct or cassette, which comprises a promoter which is derived from the same species as the host plant. In other words, the system is a DNA construct or cassette that comprises an endogenous promoter that is native to the host plant. In the homologous system the homologous promoter is preferably functionally fused in frame with a reporter gene, which may be heterologous.

The term **"heterologous system"** means a DNA construct or cassette, which comprises a promoter, which is exogenous to host plant species. The host plant in its native condition does not comprise said promoter. In other words, the promoter is not native to the host plant and the host plant must be transformed with a foreign promoter, which is derived from another organisms. In the heterologous system the heterologous promoter is preferably functionally fused in frame with a reporter gene, which may be heterologous or homologous.

The term **"homologous gene, protein or gene product"** means that the gene is endogenous to the host plant species. In other words, the gene is native to the host plant species, and non-transformed host plant species also produce said proteins or gene products.

The term **"heterologous gene, protein or gene product"** means that the gene is exogenous to the host plant species. The untransformed plant does not produce said protein. In other words, it is not native to the host plant species, and the host plant must be transformed with a foreign gene, which is derived from other organisms, before it can produce the heterologous protein or gene product.

The term **"zero generation"** means the transformed plant. Seed from said **"zero generation plant"** may be directly used for production of the desired gene products from the germinating seeds or seedlings during cotyledon development, but they can also be used for providing further or subsequent generations of plants, comprising at least one of DNA construct or cassette carrying at least one member of the family of the Rubisco promoters including SEQ ID NO:1 or SEQ ID NO:3 or combinations thereof. The seeds of said plants, which have been checked and selected so that they carry at least one DNA construct are preferably used for production purposes.

### General Description of the Invention

The present invention is related to transgene expression in germinating seedlings and sprouts. According to the present invention a strong protein expression is achieved by fusing the gene coding for the desired gene products with novel Rubisco promoters cloned from *Brassica rapa*. The novel promoters are selected from a group of Rubisco promoters derivable from *rbcS* genes, which have been selected from *rbcS* genes, which are abundantly expressed in light-grown cotyledons of *Brassrca*.

The novel promoters comprise the family of Rubisco promoters, which include SEQ ID NO:1 or SEQ ID NO:3. The promoter sequences are derived from isolated native Rubisco promoters, but similar sequences can be prepared by other means including synthetic and semisynthetic methods.

The Rubisco promoters are useful for designing recombinant DNA constructs or expression cassettes comprising SEQ ID NO:1 or SEQ ID NO:3 functionally fused in frame with reporter genes encoding desired gene products.

The promoters of the present invention were obtained by selecting and identifying genes, which were highly expressed in the developing cotyledons of light grown seedlings of *Brassica* species. 3'-UTRs of said highly expressed genes were isolated and characterized. Using said method three strong Rubisco promoters, characterized by having the sequences SEQ ID NO:1 or SEQ ID:3 and the capability of directing gene expression in a spatiotemporal manner in developing cotyledons. Said two promoters of the family of Rubisco promoters of the present invention were used for designing DNA constructs or expression cassettes, which comprise at least one, optionally two or more of said Rubisco promoters functionally linked to a gene or nucleic acid sequences encoding a heterologous protein or gene product. Usually, heterologous gene products are produced, but the promoter may as well be functionally combined to genes encoding homologous proteins or gene products. Thereby, a host plant may be transformed with several copies of a native gene and thereby the yield of an industrially important, desirous, native protein may be increased. In some cases, in order to further improve the gene expression, one or more of said promoters, in any combination, may be inserted in tandem fashion into the DNA construct or expression cassette.

The preferred reporter genes of the present invention are the *uidA* gene encoding GUS, which is an easily detectable marker protein. Another exemplified "reporter gene" is the gene coding for human serum albumin (HSA). Useful reporter genes may be designed synthetically from selected parts of immunoglobulin Ig(G) heavy chain combined with sselected parts of medically active proteins. Such a synthetic gene consisting of part of immunoglobulin (Ig) G heavy chain and extra cellular domain of Tumor Necrosis Factor Receptor (TNFR) with or without an ER-retention signal KDEL, and an antibody reporter gene comprising the heavy and/or light chains of human antibodies directed against hevein 1C2 antigen are used to exemplify the feasibility of the invention. The "reporter genes" listed above are only examples and it is evident to a person skilled in the art that the DNA constructs or cassettes comprising the promoters of the present invention may be functionally fused in frame with any other reporter gene coding for a desired product.

The recombinant constructs or expression cassettes of the present invention comprising one or more endogenous promoters, representing the homologous system according to the present invention or one or more exogenous promoters, representing the heterologous system according to the present invention, were used for transforming homologous and heterologous plants, respectively. Blant species are exemplified by *Brassica sp, Nicotiana tabacum* and *Camelina sativa*. Plant transformation procedures are familiar to those skilled in the art and therefore any other plant species can be transformed as well with the constructs according to the present disclosure. Applicable transformation systems include, but are not limited for example to the conventional *Agrobacterium* mediated transformation system. Especially transformation of *Camelina* plants according to a novel transformation system described in WO 02/38779 and US 20040031076 corresponding to US10/416,091 may be used.

The host plants were transformed with one or more of the above described DNA constructs or expression cassettes. Seeds from the transformed host plants, representing a zero generation, are collected and used for production of subsequent plant generations providing the transgenic seeds, which are used in the production of the desired proteins or gene products by allowing said seeds to germinate in contained conditions, for example on buffered agar plates or in aerated vessels, such as appropriate fermentation equipment. The transformed seed provides an excellent nutritional source and the transformed seed may germinate into seedlings in a solution comprising mainly water, which may be appropriately buffer and contain growth hormones and other advantageous growth and germination promoting ingredients. Such cultivation enables production under sterile conditions and an easy recovery of the gene products.

The following examples are various embodiments of the present invention.

### EXAMPLE 1. Rubisco mRNA types expressed in cotyledons of germinating Brassica rapa (campestris) seeds

A cDNA library was constructed in order to identify the most abundant types of Rubisco mRNA to be expressed in cotyledons of germinating *Brassica rapa* (*campestris*) seeds.

Total RNA was isolated from 4-days old *Brassica* seedlings, and a mRNA fraction was isolated from the total RNA preparations using oligo(d)T cellulose. A first strand cDNA was synthesized using oligo(d)T with M-MLV (Point mutant) reverse transcriptase. The next PCR step was carried out with a forward primer e3a
5'-CAUCAUCAUCAUCAACCGTCAAGTCCAGTGCATCAGTTTCAT-3'(SEQ ID NO:4) specific to the 3^{rd} exon of Rubisco SSU coding region and the reverse primer atu
5'-CUACUACUACUATTTTTTTTTTTTTTT-3' (SEQ ID NO: 5), an oligo(d)T derivative, specially designed according to CloneAmp procedure (Life Technologies). Both primers comprised on their 5'-terminal end several dUMP residues, which were destroyed by the enzyme UDG (Uracil DNA Glycosylase). The PCR step was carried out in 2 cycles, and subsequently the PCR product was digested by UDG and directly inserted into the linearized pAMP1 vector (Life Technologies) containing special protruding 3'-terminal ends compatible with protruding 3'-terminal ends of the RT-PCR products. The insert containing vector was transformed into competent *E.coli* strain XL-1. One hundredplaques were selected and analyzed. Inserts from plasmid DNA were amplified by PCR and resulting PCR-products were sequenced. Relative number of separate colonies containing inserts of each type was calculated by the aid of sequence analysis.

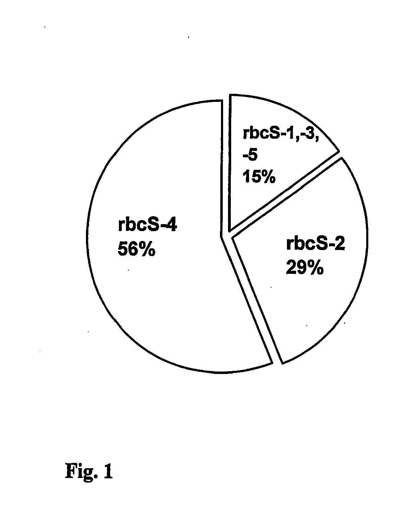

Referring to Fig. 1 sequence analysis of the cloned 3'UTRs showed striking quantitative differences between different Rubisco mRNA species. The sequence named '56' comprised 56% of all the Rubisco mRNA cloned. The other sequence named '29' comprised 29% of all the Rubisco mRNA. The rest 15% of the clones corresponded to the other types of Rubisco mRNAs.

The 29-type and 56-type sequences received from the cDNA library were compared to published sequences. The sequence alignments indicated that these Rubisco mRNAs are expressed from novel Rubisco promoters. The 29-type sequences are called *rbcS-2* and the 56-type sequences are called as *rbcS-4*, respectively.

### EXAMPLE 2. Cloning Rubisco promoters obtained from Brassica rapa

Based on the '56' and '29' type of sequences reverse primers were designed to be used in subsequent steps of promoter cloning.

### Cloning of rbcs-2 promoter

An EST-library was constructed first. The most common type of UTR found was UTR2. This UTR-2 was used to design reverse primers for Genome Walking step. Genomic DNA of *Brassica rapa* was digested by *EcoRV, DraI, HincII, PvuII, SmaI* and *SspI* and ligated to adapters
(5'-GTAATACGACTCACTATAGGGCACGCGTGGTCGACGGCCCGGGCTGGT (SEQ ID NO:6) and 5'-p-ACCAGCCC-NH₂ _3'(SEQ ID NO:7)
to get six DNA libraries.

The next PCR amplifications (first and nested) were performed with adapter primer AP1
5'-GTAATACGACTCACTATAGGGC-3' (SEQ ID NO:8) and UTR2-specinc L1 primer
5'-GGCCACACTTGACAATCCGATATAACATGCCTCA-3' (SEQ ID NO:9).

Nested PCR was conducted with AP2 primer
5'-ACTATAGGGCACGCGTGGT-3' (SEQ ID NO:10) and nested UTR2-specific L2 primer
5'-CAAATGGAAATGAAATGAGGTAG-3' (SEQ ID NO:11).

The longest 900 bp product was obtained by using a *DraI* DNA library. This fragment was cloned into a pGEM3Zf(+) vector and sequenced. The sequence was compared with the sequences in GenBank database. The most homologous sequence found was *B. napus rbcS* (accession number X61097).

Near the 5'-end of one of the clones received (*Rud3*) was a 22 nt long stretch lacking from *B.napus rbcS* (beginning from 1037 nt of *B.napus rbcS*). Two reverse primers, *RbNco* and *RbSiB,* downstream from the putative transcription initiation site (based on the homology with X61097) and two forward primers, *BNRb1* and *BNRb3,* based on X61097 homology, were designed. Full-length *rbcS*-*2*-gene was amplified using *BNRb1* as a forward primer and UTR2-L2 as a reverse primer. Subsequently, two promoters of different length were amplified in nested PCRs using combinations of *BNRb3* as a forward primer and *RbSiB* (with signal peptide) as a reverse primer, or *BNRb3* as a forward primer and *RbNco* as a reverse primer (without signal peptide).

### Cloning of rbcS-4 promoter

Promoter cloning was conducted in several steps. Two reverse primers (for the first and nested PCRs) matching with the same sequences on the beginning of the first exons of three published Rubisco genes were used for the first step of Genome Walking.

Genomic DNA was isolated from *Brassica rapa* leaves and divided into six fractions. Each fraction was digested by one of six restricting enzymes (*EcoRV, DraI, PvuII, StuI, SspI, XmnI*) and ligated with Genome Walking adapters (Clontech) mentioned above. Each restriction-ligation mixture represents a genomic DNA library.

The next step included two successive PCRs (first and nested) using adapter-specific AP1 and AP2 (forward) and gene-specific (reverse) primers. The PCR was started by using three different reverse primers, annealed to different parts of the first exon of Rubisco SSU gene in order to get the overlapping PCR products listed below.
(RbcS-RN: 5'-ACCCGGGCCCAGGAGAGCATAGAGGAAGCC-3' (SEQ ID NO:12),
RbcS-R1: 5'-CGGTGAATGGAGCGACCATCGTGGCTTGAG-3' (SEQ ID NO:13),
RbcS-R2: 5'-CTGTGAATGGAGCAACCATGGCCGCTTGAG-3' (SEQ ID NO:14).

The six genomic DNA libraries described above produced amplification products after nested PCR. These products were directly cloned into pGEM-T-Easy vector (Promega) using TA-cloning. Colonies were screened using PCR with M13-universal and reverse primers. Colonies carrying plasmid DNA with insert were grown in liquid cultures and plasmid DNA isolated was used for sequencing analysis.

A total number of about ninety plasmid DNA insert-containing clones were analyzed. Based on data obtained from the sequencing analysis the sequences were divided into five groups according to sequence similarities. Three promoters were identified to be similar to the ones published in GenBank. Moreover, PCR using specially designed forward primers, specific to the cloned promoter regions, and reverse primers, specific for the '56'- type of 3'UTR (*rbcS-4* type of 3'UTR) allowed identification of putative promoters having the 56 type of 3'UTR (*rbcS-4* type of 3'UTR) in the genome. This promoter was called '56A'.

Based on the obtained sequences new reverse primers were designed to make next PCR set with the same forward primers (AP1, AP2) and the new reverse primers and using the same genomic DNA libraries. This procedure was repeated 4 times. After the fourth PCR cycle the resulting sequences allowed the designation of promoter-specific forward primers. The reverse primer was designed to include a special site for *BpiI* to create a *NcoI*-compatible restriction site. PCR using these primers and HiFi KOD polymerase enabled identification of the '56' type of promoter *rbc-4A* (SEQ ID NO:1) among other sequences. By means of Genome Walking techniques another promoter with the '56' type 3'UTR was found bound in the genome. This *rbcS-4B* promoter (SEQ ID NO:2) was 98% similar to *rbcS-4A* on the length of about 230 nt region in (1953-2175 nt SEQ ID NO:1 and 794-1016 nt in SEQ ID NO:2), but distal parts of *rbcS-4A* and *rbcS-4B* showed less than 40% similarity. Figure 2B gives an alignment of -267 to +33 nt regions of these two promoters. *rbcS-4B* promoter was also cloned with a proof-reading KOS polymerase and its functional activity was studied further.

Using the same approach, totally four steps of Genome Walking were applied to clone the *rbcS-4A* promoter (SEQ ID NO:1) and two steps were applied to clone *rbcS-1, rbcS-3* and *rbcS-5* promoters (SEQ ID NO:21 SEQ ID NO:22, and SEQ ID NO:23, respectively). After the final step of Genome Walking whole length promoters were cloned using the proof-reading Pfu enzyme. The 3'-terminal ends of the cloned promoter sequences were designed so that they can be ligated with reporter genes. Promoter sequences obtained have been analysed using Genbank BLAST system. There have been identified *Brassica* promoters, similar (up to 98-99%) to rbcS-3 and rbcS-5 promoters, having the accession.numbers X55937 and X75334, respectively. All the promoters cloned and known were compared to each other by computer alignment program. This analysis showed that all the promoters have more or less similar parts located mostly in about 300 nt region. An alignment of 300 bp length proximal parts of these *rbcS* promoters (excluding *rbcS-4B* (see below)) is presented in Fig.2A (rbcS-2 is SEQ ID NO:3; rbcS-4A is SEQ ID NO:1). The Genome Walking data showed that there were two partially different rbcS-4 (called rbcS-4A and rbcS-4B) promoters connected to the same 3'UTRs and being very similar on the last 230 bp on their 3'-terminal ends (Fig.2B) (rbcS-4A is SEQ ID NO:1; rbcS-4B is SEQ ID NO:2). On the other hand, the resting (distal) parts of the promoters show the same low level of homology (40%) as they show in alignment with other Rubisco promoters.

Alignment of one of the published *Brassica napus* Rubisco promoter (accession number X61097) with *rbcS-2* (SEQ ID NO:3), demonstrates some differences (91% similarity) between them (Fig.2C). There are also differences in 3'UTR regions. Therefore these two promoters are not the same ones and probably diverged during evolution or selection process of *rbcS* gene family in *Brassica* species.

Referring to Figure 3 alignment of *rbcS-4A* (SEQ ID NO:1) promoter sequence and published *Brassica* rubisco promoter (X61097) revealed dissimilarity of 52%.

Similarly, referring to Figure 4 alignment of *rbcS-4A* promoter sequence with the published *Chrysanthemum rbcS-1* promoter (AY163904) revealed dissimilarity of 57%.

Markedly, there are only three stretches in *rbsC-4*A promoter (1007-1440 nt, 1776-1950 nt and 1959-2175 nt of SEQ ID NO:1) that have a quite high homology (similarity of about 93%) with *Brassica* genome project database (Fig. 17). Accession number BH484651 represents genomic clone of *Brassica oleracea* and CD811761 represents cDNA clone of *Brassica napus*. No other parts of the *rbcS-4A* promoter sequence are found in any database including *Brassica* genome project.

Clearly the nucleotide sequences of *rbcS-2* (SEQ ID NO:3) and *rbcS-4A* and B (SEQ ID NO: 1 and 2, respectively) are novel and useful as described in this disclosure.

### EXAMPLE 3. Fusion- constructs rbcS-4A-GUS, rbcS-4A-HSA, rbcS-4B-GUS, rbcS-2-GUS, rbcS-2-HSA, rbcS-2-Ab(L+H)-1C2, rbcS-4A-Ab(L+H)-1C2 , rbcS-2-TNFR-Fc and rbcS-4A-TNFR-Fc

The promoters were amplified with reverse primers to get *NcoI*-compatible restriction site on their 3'-terminal ends. Vector pCAMBIA1301 (CAMBIA) containing GUS gene with *NcoI* site on its 5'-terminal end was used. HSA fusion constructs were designed in a pBIN19-based plasmid *pGPTV* with an inserted HSA gene. A sequence of a codon-optimized HSA gene with an artificial polyA signal was added as shown in Figure 19. *RbcS-4A* and *rbcS4B* were cut out using *BpiI, HindIII. RbcS-2* was cut out using *NcoI, HindIII*. The promoters *RbcS 4A, rbcS-4B*, and *rbcS-2* were cloned into pCAMBIA1301 or pGPTV vectors opened by *NcoI, HindIII*. The terminators used for these constructs were as follows: nos-terminator in GUS-containing pCAMBIA1301 vector, and *rbcS-4* type of 3'UTR plus part of known *Brassica rapa rbcS* terminator from GenBank was used in HSA-containing pGPTV plasmids.

Constructs Rbcs-2-Ab(L+H)-1C2 and RbcS-4-Ab(L+H)-1C2 contain the same antibody regions and the same terminator (polyA) signal from the natural *Brassica* rubisco *RbcS-4* gene (directly from the genome). The antibody protein molecule was originally developed against hevein 1C2 antigen. RbcS-2-Ab(L+H)-1C2 consists of *RbcS-2* promoter, light chain(anti-hevein 1C2) coding region as shown in Figure 20, RbcS-4 terminator (SEQ ID NO:17) as shown in Figure 21, another *RbcS-2* promoter, heavy chain (anti hevein 1C2) (SEQ ID NO:18) coding region as shown in Figure 22 and another Rbcs-4 terminator (SEQ ID NO:20). The RbcS-4-Ab(L+H)-1C2 construct consists of *Rbcs-4* promoter, light chain (anti-hevein 1C2 (SEQ ID NO:16) coding region, RbcS-4 terminator, another *RbcS-4* promoter (SEQ ID NO:2), heavy chain (anti-hevein 1C2) (SEQ ID NO:18) coding region, and another RbcS-4 terminator (SEQ ID NO:20).

For the constructs Rbcs-2-Ab(L+H)-1C2 and RbcS-4-Ab(L+H)-1C2 *rbcS-2* and *rbcS-4* promoters were cut by *SalI, HindIII* and ligated with pVK1-CHC(constant heavy chain)-rbcS-4-terminator, digested with *SalI*, and *HindIII providing the pVK1-RbcS-2(Rbcs-4A)*promoter - *CHC-RbcS-4-terminator*. RbcS-4 terminator was originally cloned with CHC by *BsiWI, EcoRI*. Variable heavy chain region of 1C2 antibody (VH-1C2) was cut out by *BpiI, Bsp120I* and cloned into a pVK1-Rbcs-2(Rbcs-4)-promoter-CHC-RbcS-4-terminator vector by the same sites. The resulting plasmid was the plasmid containing whole H (heavy) chain unit. The same strategy was used to get the whole L (light) chain unit. L chain unit was then cloned into pCAMBIA1301 vector from where *35S-gusA* or *35SUidA* gene was removed. This provided pCAMBIA1301-L-chain. In the final step the H-chain unit was inserted into pCAMBIA1301-L-chain vector to get the final pCAMBIA1301-H-L. The plasmid was used for plant transformation using *Agrobacterium* mediated strategy. Ig-TNFR (ENBREL) construct contains *rbcS-2* or *rbcS-4* promoters, TNFR (tumor necrosis factor receptor) part (489 nt) as shown in Figure 23 (SEQ ID NO:19) comprising the Ig CHC part (CH2 and CH3 domains) and terminators. TNFR part was cloned directly from human mRNA by reverse transcription followed by PCR, ligated into pGEM-T-Easy plasmid by TA-cloning procedure and sequenced from both directions with M13-universal and reverse primers. Ig CHC part was obtained by PCR and sequenced thereafter. Cloning strategy included ligation of Ig CHC part by *BsiWI* site and introducing promoter into pVK1 plasmid (pUC19 derivative), containing a *rbcS*-terminator. Then TNFR part digested by *BsmB1* was introduced into this plasmid, and whole the insert was re-cloned into big pCAMBIA1300 or pCAMBIA2300 plasmids.

IgCHC part was obtained in two variants. The first was without any changes in its 3'end and the second one contained KDEL signal in its 3'end. This signal is 12 nt long sequence AAAGACGAGCTG (SEQ ID NO: 24) and is introduced just before the STOP-codon. Several terminators were used in the Ig-TNFR constructs. One was rbcS-4 terminator (about 500 nt) being the same as used in antibody constructs. Another terminator was a longer version of the rbcS 4-terminator (being about 2kb). Still another terminator used was from *Arabidopsisis VSP1* (vegetative storage protein-1 gene), the part situating right behind the STOP codon and before cleavage site was used and was connected with part of rbcS- 4 terminator (SEQ ID NO:17) shown in Figure 24. In some of the constructs one or two MAR (matrix attachment regions) sequences (about 2kb) were also introduced. In case the construct contained two MAR sequences they were introduced before the promoter and after the terminator.

### EXAMPLE 4. Plant transformation

To exemplify the functionality of the novel promoters according to this disclosure, we transformed plants of *Brassica* species, *Nicotiana tabacum* plants and *Camelina sativa* plants. One skilled in the art is able to transform plants of other species.

*Brassica* plants were transformed with *A. tumefaciens* strain LBA4404 carrying the pCAMBIA1301 or pGPTV-HPT binary vectors by leaf disk inoculation. Tobacco plants *Nicotiana tabacum* cv. Samsung were transformed with *A. tumefaciens* strain LBA4404 carrying pGPTV-HPT binary vectors by leaf disc inoculation. Putative transformants were selected on 30 mg/l hygromycin. Positive lines were transferred to the greenhouse for further studies

*Camelina* plants were transformed with *A. tumefaciens* strain C58 (helper plasmid pGV3850) carrying the pCAMBIA1300 binary vectors by leaf disc inoculation. Putative transformants were selected on 20mg/l hygromycin. Positive lines were transferred to the greenhouse for further studies.

### EXAMPLE 5. Quantitative GUS Assay for expression analysis

The assays were carried out with tobacco leaves or *Camelina* seedlings. Fresh plant material was mechanically disrupted in Tris-buffer, containing 2-ME. Protein concentrating in extracts was determined using Bio-Rad assay. GUS activity was determined in spectrophotometer using methyl-umbelliferyl as a substrate for the enzymatic reaction. Incubation was 30 min at +37° C and developed color was measured in spectrophotometer at 450 nm wavelength. Non-transgenic plants were used as negative controls.

### EXAMPLE 6. Expression analysis of mRNA with Real-Time RT-PCR and Northern analysis

Total RNA isolated from cotyledons of germinating *Brassica* or *Camelina* seeds or tobacco leaves were reverse transcribed with gene specific reverse primers. The reverse primers were designed for non-similar parts of all the 3'UTR known as well as for HSA, GUS, heavy and light chains of anti-hevein 1C2 antibody and the third exon of Rubisco SSU coding region. cDNA obtained was used for Real-time PCR step using forward and the same reverse primers.

Real-Time procedure was conducted on API7000 machine mainly according to the manual using SYBRgreen quantitative variant of the method. The passive reference dye was ROX. The calibration curves were constructed using PCR products amplified from genome and purified with the same primers as in Real-Time process. The result was expressed in number of molecules per 1 ng of RNA sample originally taken.

For the Northern analysis total RNA was isolated from plant material and run on agarose gel and transferred on to the membrane. Then RNA was cross-linked to the membrane by short exposure to UV light. Next step is hybridization with specific RNA probe, synthesized in vitro from bacterial T7 or SP6 promoters. Hybridization was going on overnight at optimal temperature, specially optimized for every probe. After washing the membrane is undergone to incubation with antibodies recognizing DIG-labels on the probe. The amount of the RNA probes (i.e. specific mRNA) was detected by enhanced luminescence using negative and positive controls (varying concentrations), allowing the determination of the amount of specific mRNA in the experimental sample.

### EXAMPLE 7 Expression level of Rubisco genes and total Rubisco mRNA in germinating seeds increases toward end of cotyledon development

The total RBCS mRNA content in constant light conditions increased during the first 3-4 days and remained on a high level for the next 5 to 7 days (Figure 16).

In order to determine the expression levels of different Rubisco genes and also total Rubisco mRNA production in germinating *Brassica napus* seeds we measured the amount of total Rubisco mRNA in seeds on 0, 1, 2, 3 and 4 day of germination in constant light conditions by Real-Time PCR. This is illustrated in Fig. 9.

The quantitative data shown in Figure 9 (first column ), demonstrates the amount or number of Rubisco mRNA molecules in 1 ng of total mRNA per an average sample. Clearly, the amount of mRNA molecules increased from day 0 to day 4, showing the highest amounts on the 4^{th} day. On 4^{th} day of germination the amount of RBCS mRNA determined in most of the samples was about 4-7 x 10⁷ molecules per 4 ng of total mRNA.

### EXAMPLE 8. rbcS-4 type of RBCS mRNA is the most prevalent and active type of mRNA at the stage of germinating seeds of plant development

The amount of different types of RBCS mRNAs was analyzed by the Real Time process described above. The expression levels of *rbcS-2, rbcS-3, rbcS-4* and *rbcS-5* were determined on 0-4^{th} day of *Brassica napus* seed germination by using primers specific to non-similar parts of 3'UTRs of those mRNA species (Fig.*5*). Forward primers were designed so that they have longer right part, corresponding to specific 3'UTR type. The shorter left part of each primer corresponds to the end of the RBCS coding region. This left part helps to increase length and therefore Tm of the primer, but does not disturb the specificity of it (Fig.5).

Data summarized in Figure 9 (columns 3-6), demonstrates dramatic differences in the expression levels. The most abundant type during the four days of seed germination was *rbcS-*4 RBCS mRNA, but as we have already noticed above there are at least two *rbcS* genes driven by the partially similar promoters (*rbcS-4A* and *-B*; SEQ ID NO: 1 and SEQ ID NO:2, respectively) and connected to the same 3'UTR (*rbcS-4* type of 3'UTR). This may mean that each *rbcS-4* gene can contribute to the sum activity of the gene. But according to quantitative GUS expression data obtained from *rbcS-4B*-GUS transgenic tobacco plants (Fig. 10) the activity of the promoter is very low and doesn't seem to have remarkable influence on total amount of mRNA containing *rbcS-4* type of 3'UTR.

The data presented here clearly demonstrates the prevalence of *rbcS-4* type of RBCS mRNA on the later stage of germinating seeds of plant development.

Referring now to results shown in Figure 9 Real-Time PCR showed that ***r**bcS-4* promoter was more active at the fourth day of germination than any other Rubisco promoter examined. The expression level of different RBCS genes followed different kinetics, for example, at third day rbcS-2 (SEQ ID NO:3) and rbcS-3 (SEQ ID NO:21) were more active than rbcS-4 (SEQ ID NO:22) and rbcS-5 (SEQ ID NO:23). These characteristics are extremely important when selecting a promoter for a production method of foreign proteins or other desired gene products to be produced in germinating seeds or sprouts.

Unstable transgenic proteins may degrade quite fast because of enhanced protein mobilization capacity of plant cells in tissues of germinating seeds. When using a promoter such as *rbcS-4* with delayed kinetic of activity, there are more chances to protect accumulation of transgenic protein product from the action of lytic vacuoles. Moreover, additional benefits of using *rbcs-4A* in transgenic constructs arise from the fact that this is the strongest promoter out of the four promoters analyzed at later stages of seed germination.

Figure 13 compares the accumulation of HSA mRNA in germinating *B. napus* seeds transgenic for rbcS-4-HSA or for (rbcs-2-HSA)x2. (rbcS-2-HSA)x2 is a variant of rbsS-2-HSA where 2 units of *rbcS-2* are arranged in tandem. It is evident that HSA mRNA begins to accumulate earlier in the seeds transgenic for (rbcS-2-HSA)x2.. On the other hand HSA mRNA in rbcS-4-HSA transgenic plants starts to accumulate later but the amount accumulating is somewhat bigger. As can be seen from Figure 9. the kinetics of *rbcs-4* promoter activity is more delayed than that of *rbcS-2*, and therefore it is evident that both of the promoters are functional even when in non-native conditions. In this connection native conditions mean normal non-transgenic plants, containing rubisco promoters plus rubisco genes placed in proper sites of the genome. Non-native conditions mean artificial situation, like in our case, the *Brassica* rubisco genes plus foreign reporter genes inserted in occasional places of the genome.

### EXAMPLE 9. Heterologous and homologous transgenic plants harboring rbcS-2 and rbcS-4 promoters for production of desired gene products

*rbcS-2* (SEQ ID NO: 3) and *rbcS-4* (SEQ ID NO: 1 and SEQ ID NO: 2) promoters were used for plant transformation experiments with *Brassica,* tobacco and *Camelina* plants to determine the 'promoter strength' and also to compare the expression levels in homologous and heterologous systems (i.e. plants transformed with a construct containing a promoter from the same or a different species).

The promoters were amplified with reverse primers to get *NcoI*-compatible restriction site on their 3' ends. pCAMBIA1301 vector (CAMBIA) containing GUS gene with *NcoI* site on its 5' end designed as described in Example 2 were used.

Promoters *rbcS-2* (SEQ ID NO:3) or *rbcS*-4A and B (SEQ ID NO:1 and 2) containing constructs inserted in the genome of *Brassica* represent homologous system, and the insertion of the same constructs in tobacco and *Camelina* plant' s genome represent heterologous system. Recombinant constructs containing *rbcS-2* or *rbcS-4* promoters fused in frame with reporter genes were designed as described in Example 3 and transformed into plants as described in Example 4.

mRNA expression data of transgenic *Brassica* plants containing *rbcS-4A-GUS* or *rbcS-2-GUS* is presented in Figure 7. The mRNA expression level of reporter gene was measured from cotyledons of seeds of transgenic *Brassica* plants, germinated for 4 days.

In the table shown in Figure 7 it is demonstrated that
1) expression of transgene (GUS) mRNA in both plant transformants is about 5-6 times less than the expression of corresponding native rubisco gene (rbcS-2 or rbcS-4A);
2) expression level of native rbcS-4A gene in transgenic plant correspond to the one in non-transgenic plant (Fig.9), but the expression level of native rbcS-2 gene in rbcS-2 transgenic plant looks less than the one in non-transgenic plant.
This result may point out some properties of two promoters, namely, more silencing dependency of rbcS-2 promoter in homologous plant.

For tobacco transformation experiments *rbcS-2-HSA* and *rbcS-4A*-HSA constructs were used and 7 HSA-producing plant lines for each of them was received. The mRNA expression level of HSA gene determined on 5th day of transgenic tobacco seed germination demonstrate about the same level of expression in both types of these plant lines (Figure 8). The tobacco transformation experiments show clearly that there is no significant difference between the *rbcS-2* and *rbcS-4* promoters strength, but both of them are expressing in heterologous system.

Transgenic Camelina and tobacco plants harbouring RbcS-2-GUS, RbcS-4-GUS, Rbcs-2-TNFR-Fc-56UTRshort, Rbcs-2-TNFR-FcKDEL-56UTRshort, Rbcs-4-TNFR-Fc-56UTRlong, Rbcs-4-TNFR-FcKDEL-56UTRlong were obtained and analysed. The results are shown in Figure 18A and B as Tables. Determination of GUS-activity demonstrates enzyme activity level in rbcS-2 (rbcS-4)-GUS transgenic plants to be bigger than in plants carrying conventional 35Sp-GUS construct used as positive control. Northern data are available for some TNFR-Fc-harbouring Camelina and tobacco plants. The expression level (for rbcs-4-TNFR-Fc) is comparable with that one of native rbcS genes (in Brassica about 50-100 pg/µg of total RNA for whole RBCS gene family).

### Example 10. Protein expression in transgenic plants

A construct comprising GUS gene coding region was linked to the Rubisco promoter *rbcS-4A* and transformed into an oilseed rape (*Brassica rapa*) plant using *Agrobacterium* mediated transformation. Transgenic plants were grown in greenhouse until seeds were produced. Seeds of transgenic plants were allowed to sprout in 20 °C aerated water, 24 °C aerated 20mM KNO₃ water or in 30 °C aerated water. After variable times of cultivation expressed GUS protein was isolated from the sprouts by homogenization in appropriate buffer and centrifugations. Specific GUS activity was determined by spectrophotometer (Fig. 11). Clearly, GUS activity per sprout was highest after 72 hours of cultivation using KNO₃ in the growth medium.

Protein expression of transgenic *Brassica napus, Camelina sativa* and tobacco plants carrying HSA under the control of *rbcS-2* or *rbcS- 4* was also analyzed. Similarly plants carrying tandem construct of *RbcS-2-HSA* were analyzed. Protein expression was analyzed from sprouts that germinated at constant light and 24 °C temperature for 4days. Figure 15 shows the data as % of total soluble protein. It is evident that plants carrying the tandem construct have higher expression levels of the protein than plants carrying single construct. Furthermore, it is evident that protein expression is higher under *rbcs-4* promoter than under *rbcS-2* promoter. The tandem construct having two *rbcS-2*-HSA constructs is an example of a multiple construct according to the present invention and one skilled in the art would be able to transform plants with more than two constructs in tandem as well. Similarly, one skilled in the art would be able to use tandem constructs having *rbcS-4A* as the driving promoter to obtain higher protein contents.

Protein expression of transgenic *Camelina sativa* and tobacco plants carrying TNFR constructs were analyzed. The results are shown in Fig 18.

### Example 11. In order to provide maximal activity the RbcS promoter has to be of full length

Truncated versions of *rbcS-2* promoter were cloned, (0.3 and 0.6 kb length) in fusion constructs with reporter *uid A* gene. Tobacco plants were transformed by *Agrobacterium* carrying these constructs and the GUS activity was measured from leaves of adult tobacco plants. The data obtained was compared to data obtained from the analysis of high-expressing adult tobacco plants carrying *rbcS-2* (1,6 kb) or 35S promoters connected to the GUS gene. The results as shown in Figure 15 clearly demonstrate decrease of registered GUS activity due to reduction of the length of the promoter. Therefore, it is evident that that distal regions of the *rbcS-2* promoter contain essential regulatory elements supporting basal (not-inducible) promoter activity. Comparative analysis in silico of known tomato rbcS1 promoter and our cloned Brassica rbcS-2 and rbcS-4A promoters enables to find similar consensus regulatory elements in all of them (Fig.6). It is clear that most of the known boxes are located in the -500 -600 nt region. It could be suggested that those distal parts of the promoters may have some cryptic regulatory elements or they may participate in the promoter action because of possible occurring of MAR (Matrix Attachment Region) sites, for example, in 5' regions of rbcS-4A (computer MAR prediction analysis).

## Claims

1. A Rubisco promoter sequence for transgene expression in germinating plant seedlings and sprouts, **characterized in that** it is derivable from promoters of light grown Rubisco expressing *Brassica rapa* seedlings, and has the nucleotide sequences SEQ ID NO:1 or SEQ ID NO:3.

2. The Rubisco promoter sequence according to claim 1, **characterized in that** it has the nucleotide sequence SEQ ID NO:1.

3. The Rubisco promoter sequence according to claim 1, **characterized in that** it has the nucleotide sequence SEQ ID NO:3.

4. An expression cassette for transgene expression in germinating plant seedlings and sprouts, **characterized in that** it comprises at least one Rubisco promoter sequence according to claim 1, operably linked to a nucleic acid sequence encoding a heterologous or homologous protein or gene product.

5. The expression cassette according to claim 4, **characterized in that** the Rubisco promoter sequence is SEQ ID NO:1.

6. The expression cassette according to claim 4, **characterized in that** the Rubisco promoter sequence is SEQ ID NO:3.

7. The expression cassette according to claim 4, **characterized in that** the nucleic acid sequence encoding the heterologous or homologous protein or gene product is a naturally existing sequence or codon optimized sequence.

8. The expression cassette according to claim 7, **characterized in that** the heterologous protein or gene product is human serum albumin.

9. The expression cassette according to claim 7, **characterized in that** the heterologous or homologous protein or gene product is a medically active protein or a part thereof.

10. The expression cassette according to claim 7, **characterized in that** the heterologous or homologous protein or gene product is an antibody.

11. A transgenic plant, **characterized in that** the transgenic plant or its subsequent generations carries at least one expression cassette, which comprises a Rubisco promoter sequence SEQ ID NO:1 or SEQ ID NO:3 operably linked to a nucleic acid sequence encoding a heterologous protein or gene product.

12. The transgenic plant according to claim 11, **characterized in that** it is a transgenic *Brassica* or transgenic *Camelina sativa.*

13. The transgenic plant according to claim 11, **characterized in that** it is a transgenic *Camelina sativa*.

14. A transgenic seed derived from the transgenic plant according to claim 11, **characterized in that** it carries at least one expression cassette, which comprises a Rubisco promoter sequence SEQ ID NO:1 or SEQ ID NO:3 operably linked to a nucleic acid sequence encoding a heterologous protein or gene product.

15. The transgenic seed according to claim 14, **characterized in that** it is a transgenic *Brassica* seed or a transgenic *Camelina sativa* seed.

16. The transgenic seed according to claim 14, **characterized in that** it is a transgenic *Camelina sativa* seed.

17. A transgenic seedling derived from the germinated transgenic seed according to claim 14, **characterized in that** it carries at least one expression cassette, which comprises a Rubisco promoter sequence SEQ ID NO:1 or SEQ ID NO:3 operably linked to a nucleic acid sequence encoding a heterologous_protein or gene product.

18. The transgenic seedling according to claim 17, **characterized in that** it is a transgenic *Brassica* seedling or a transgenic *Camelina sativa* seedling.

19. The transgenic seedling according to claim 17, **characterized in that** it is a transgenic *Camelina sativa* seedling.

## Patentansprüche

1. Rubisco-Promotorsequenz zur Expression von Transgenen in keimenden Pflanzensämlingen und Sprossen, **dadurch gekennzeichnet, dass** sie von Promotoren von im Licht gezüchteten *Brassica* rapa-Keimlingen ableitbar ist, die Rubisco exprimieren, und dass sie die Nucleotidsequenz SEQ ID NO: 1 oder SEQ ID NO: 3 hat.

2. Rubisco-Promotorsequenz nach Anspruch 1, **dadurch gekennzeichnet, dass** sie die Nucleotidsequenz SEQ ID NO: 1 hat.

3. Rubisco-Promotorsequenz nach Anspruch 1, **dadurch gekennzeichnet, dass** sie die Nucleotidsequenz SEQ ID NO: 3 hat.

4. Expressionskassette zur Expression von Transgenen in keimenden Pflanzensämlingen und Sprossen, **dadurch gekennzeichnet, dass** sie mindestens eine Rubisco-Promotorsequenz nach Anspruch 1 umfasst, die funktionell mit einer ein heterologes oder homologes Protein oder Genprodukt codierenden Nucleinsäuresequenz verbunden sind.

5. Expressionskassette nach Anspruch 4, **dadurch gekennzeichnet, dass** die Rubisco-Promotorsequenz SEQ ID NO: 1 ist.

6. Expressionskassette nach Anspruch 4, **dadurch gekennzeichnet, dass** die Rubisco-Promotorsequenz SEQ ID NO: 3 ist.

7. Expressionskassette nach Anspruch 4, **dadurch gekennzeichnet, dass** die das heterologe oder homologe Protein oder Genprodukt codierende Nucleinsäuresequenz eine in der Natur vorkommende Sequenz oder eine Codon-optimierte Sequenz ist.

8. Expressionskassette nach Anspruch 7, **dadurch gekennzeichnet, dass** das heterologe Protein oder Genprodukt menschliches Serumalbumin ist.

9. Expressionskassette nach Anspruch 7, **dadurch gekennzeichnet, dass** das heterologe oder homologe Protein oder Genprodukt ein medizinisch aktives Protein oder ein Teil davon ist.

10. Expressionskassette nach Anspruch 7, **dadurch gekennzeichnet, dass** das heterologe oder homologe Protein oder Genprodukt ein Antikörper ist.

11. Transgene Pflanze, **dadurch gekennzeichnet, dass** die transgene Pflanze oder ihre Folgegenerationen mindestens eine Expressionskassette enthalten, die eine Rubisco-Promotorsequenz SEQ ID NO: 1 oder SEQ ID NO: 3 umfasst, die funktionell mit einer ein heterologes Protein oder Genprodukt codierenden Nucleinsäuresequenz verbunden ist.

12. Transgene Pflanze nach Anspruch 11, **dadurch gekennzeichnet, dass** sie eine transgene *Brassica* oder transgene *Camelina sativa* ist.

13. Transgene Pflanze nach Anspruch 11, **dadurch gekennzeichnet, dass** sie eine transgene *Camelina sativa* ist.

14. Transgener Samen abgeleitet von der transgenen Pflanze nach Anspruch 11, **dadurch gekennzeichnet, dass** er mindestens eine Expressionskassette enthält, die eine Rubisco-Promotorsequenz SEQ ID NO: 1 oder SEQ ID NO: 3 umfasst, die funktionell mit einer ein heterologes Protein oder Genprodukt codierenden Nucleinsäuresequenz verbunden ist.

15. Transgener Samen nach Anspruch 14, **dadurch gekennzeichnet, dass** er ein transgener Brassica-Samen oder ein transgener *Camelina sativa-Samen* ist.

16. Transgener Samen nach Anspruch 14, **dadurch gekennzeichnet, dass** er ein transgener *Camelina-sativa*-Samen ist.

17. Transgener Sämling abgeleitet von dem gekeimten transgenen Samen nach Anspruch 14, **dadurch gekennzeichnet, dass** er mindestens eine Expressionskassette enthält, die eine Rubisco-Promotorsequenz SEQ ID NO: 1 oder SEQ ID NO: 3 umfasst, die funktionell mit einer ein heterologes Protein oder Genprodukt codierenden Nucleinsäuresequenz verbunden ist.

18. Transgener Sämling nach Anspruch 17, **dadurch gekennzeichnet, dass** er ein transgener Brassica-Sämling oder ein transgener *Camelina sativa*-Sämling ist.

19. Transgener Sämling nach Anspruch 17, **dadurch gekennzeichnet, dass** er ein transgener *Camelina sativa*-Sämling ist.

## Revendications

1. Séquence de promoteurs de Rubisco pour l'expression transgène lors de la germination de semis et pousses de plantes, **caractérisée en ce qu'**elle peut être dérivée de promoteurs de la Rubisco légèrement poussé exprimant des semis *Brassica rapa*, et possède la séquence de nucléotides SEQ ID NO:1 ou SEQ ID NO:3.

2. Séquence de promoteurs de Rubisco selon la revendication 1, **caractérisée en ce qu'**elle présente la séquence de nucléotides SEQ ID NO:1.

3. Séquence de promoteurs de Rubisco selon la revendication 1, **caractérisée en ce qu'**elle présente la séquence de nucléotides SEQ ID NO:3.

4. Cassette d'expression pour l'expression transgène lors de la germination de semis et de pousses de plantes, **caractérisée en ce qu'**elle comprend au moins une séquence de promoteurs de Rubisco selon la revendication 1, fonctionnellement liée à une séquence d'acides nucléiques codant pour une protéine hétérologue ou homologue ou un produit de gène.

5. Cassette d'expression selon la revendication 4, **caractérisée en ce que** la séquence de promoteurs de Rubisco est la SEQ ID NO:1.

6. Cassette d'expression selon la revendication 4, **caractérisée en ce que** la séquence de promoteur de Rubisco est la SEQ ID NO:3.

7. Cassette d'expression selon la revendication 4, **caractérisée en ce que** la séquence d'acides nucléiques codant pour la protéine hétérologue ou homologue ou le produit de gène est une séquence existant naturellement ou une séquence de codons optimisée.

8. Cassette d'expression selon la revendication 7, **caractérisée en ce que** la protéine hétérologue ou produit de gène est une albumine du sérum humain.

9. Cassette d'expression selon la revendication 7, **caractérisée en ce que** la protéine hétérologue ou homologue ou produit de gène est une protéine médicalement active ou une partie de celle-ci.

10. Cassette d'expression selon la revendication 7, **caractérisée en ce que** la protéine hétérologue ou homologue ou produit de gène est un anticorps.

11. Plante transgénique, **caractérisée en ce que** la plante transgénique ou ses générations suivantes portent au moins une cassette d'expression, qui comprend une séquence de promoteurs de Rubisco SEQ ID NO:1 ou SEQ ID NO:3 fonctionnellement liée à une séquence d'acides nucléiques codant pour une protéine hétérologue ou un produit de gène.

12. Plante transgénique selon la revendication 11,
**caractérisée en ce qu'**elle est une *Brassica* transgénique ou *Camelina sativa* transgénique.

13. Plante transgénique selon la revendication 11,
**caractérisée en ce qu'**elle est une *Camelina sativa* transgénique.

14. Semence transgénique dérivée de la plante transgénique selon la revendication 11, **caractérisée en ce qu'**elle porte au moins une cassette d'expression qui comprend une séquence de promoteurs de Rubisco SEQ ID NO:1 ou SEQ ID NO:3 fonctionnellement liée à une séquence d'acides nucléiques codant pour une protéine hétérologue ou un produit de gène.

15. Semence transgénique selon la revendication 14, **caractérisée en ce qu'**elle est une semence de *Brassica transgénique* ou une semence de *Camelina sativa* transgénique.

16. Semence transgénique selon la revendication 14, **caractérisée en ce qu'**elle est une semence de *Camelina sativa* transgénique.

17. Semis transgénique dérivé de la semence transgénique germée selon la revendication 14, **caractérisé en ce qu'**il porte au moins une cassette d'expression qui comprend une séquence de promoteurs de Rubisco SEQ ID NO:1 ou SEQ ID NO:3 fonctionnellement liée à une séquence d'acides nucléiques codant pour une protéine hétérologue ou un produit de gène.

18. Semis transgénique selon la revendication 17,
**caractérisé en ce qu'**il est un semis de *Brassica* transgénique ou un semis de *Camelina sativa* transgénique.

19. Semis transgénique selon la revendication 17,
**caractérisé en ce qu'**il est un semis de *Camelina sativa* transgénique.
